# EUROPEAN PATENT SPECIFICATION

(11) **EP 3 481 231 B1**
(45) Date of publication and mention of the grant of the patent: **01.12.2021**
(21) Application number: 17740246.8
(22) Date of filing: 06.07.2017
(51) Int. Cl.: A23L 33/00, A61K 31/702, C07H 3/06, A23K 10/28, A23L 29/30, A23L 33/10, A23L 33/21, A23C 1/12, A23C 9/12, A23C 21/02

(54) **PROCESS FOR MAKING GALACTO-OLIGOSACCHARIDE PRODUCT**
VERFAHREN ZUR HERSTELLUNG VON GALACTO-OLIGOSACCHARID-PRODUKTEN
PROCÉDÉ DE FABRICATION D'UN PRODUIT DE GALACTO-OLIGOSACCHARIDE

(30) Priority: 11.07.2016 US 201662360819 P
(43) Date of publication of application: 15.05.2019
(73) Proprietor: Proliant Dairy, Inc., Ankeny, Iowa 50021 (US)
(72) Inventor: MOZAFFAR, Zahid, Ballwin, Missouri 63021 (US); PETERSON, Mark E., Des Moines, Iowa 50312 (US)
(74) Representative: Schwegman Lundberg Woessner Limited
(86) International application number: PCT/US2017/040907
(87) International publication number: WO 2018/013402

(56) References cited:
- EP-A2- 2 252 699
- WO-A1-2006/087391
- WO-A1-2007/090894
- WO-A1-2012/160080
- WO-A1-2015/132402
- US-A1- 2010 255 147
- GEIGER BARBARA ET AL: "From by-product to valuable components: Efficient enzymatic conversion of lactose in whey using [beta]-galactosidase fromStreptococcus thermophilus", BIOCHEMICAL ENGINEERING JOURNAL, vol. 116, 5 April 2016 (2016-04-05), pages 45-53, XP029805892, ISSN: 1369-703X, DOI: 10.1016/J.BEJ.2016.04.003
- MARINA GOLOWCZYC ET AL: "Use of whey permeate containing in situ synthesised galacto-oligosaccharides for the growth and preservation of Lactobacillus plantarum", JOURNAL OF DAIRY RESE, CAMBRIDGE UNIVERSITY PRESS, CAMBRIDGE, GB, vol. 80, no. 3, 1 August 2013 (2013-08-01) , pages 374-381, XP008178708, ISSN: 0022-0299, DOI: HTTP://DX.DOI.ORG/10.1017/S002202991300035 6NOSUBSCRIPTION
- FISCHER CHRISTIN ET AL: "Synthesis of galactooligosaccharides using sweet and acid whey as a substrate", INTERNATIONAL DAIRY JOURNAL, vol. 48, 24 January 2015 (2015-01-24), pages 15-22, XP029170303, ISSN: 0958-6946, DOI: 10.1016/J.IDAIRYJ.2015.01.003
- HUGO AYELÉN A ET AL: "Whey permeate containing galacto-oligosaccharides as a medium for biomass production and spray drying ofLactobacillus plantarumCIDCA 83114", LWT- FOOD SCIENCE AND TECHNOLOGY, vol. 69, 16 January 2016 (2016-01-16), pages 185-190, XP029444813, ISSN: 0023-6438, DOI: 10.1016/J.LWT.2016.01.031
- NATH ARIJIT ET AL: "Synthesis of Lactose-Derived Nutraceuticals from Dairy Waste Whey-a Review", FOOD AND BIOPROCESS TECHNOLOGY ; AN INTERNATIONAL JOURNAL, SPRINGER-VERLAG, NEW YORK, vol. 9, no. 1, 11 September 2015 (2015-09-11), pages 16-48, XP035950723, ISSN: 1935-5130, DOI: 10.1007/S11947-015-1572-2 [retrieved on 2015-09-11]
- IHLI J ET AL: "Effect of galacto-oligosaccharide concentration on the kinetics of lactose crystallisation", INTERNATIONAL DAIRY JOURNAL, ELSEVIER APPLIED SCIENCE, BARKING, GB, vol. 41, 5 October 2014 (2014-10-05), pages 26-31, XP029095679, ISSN: 0958-6946, DOI: 10.1016/J.IDAIRYJ.2014.09.001

## Description

### BACKGROUND

Many dairy product production processes result in byproducts. For example:
1) Cheese production results in the formation of dairy byproducts, such as whey byproducts. Whey can be further processed to remove some components, most notably a portion of the protein, to form a dairy byproduct usually referred to as whey permeate.
2) Milk protein concentrate production results in the formation of dairy byproducts, most notably milk permeate.

It has become common to attempt to convert dairy byproducts, such as whey permeate or milk permeate, to other useful nutritional products with a higher potential profitability than milk permeate and whey permeate.

An example of a potentially higher-profit product are oligosaccharides, which have been used more and more as additives for human food or animal feed due to potential digestive health and immunity enhancing capabilities. Galacto-oligosaccharides (GOS), one type of oligosaccharide, are prebiotic compounds that can be utilized by probiotic bacteria in the small and large intestines to improve intestinal microflora and other health benefits including improved mineral absorption during digestion. GOS can also act as a soluble fiber that can provide flavor enhancement, moisture retention, and shelf-life extension for food products. Soluble fiber, including GOS, can also be used as a binding agent for food products, such as granola, cereal, or food bars.

WO2012/160080 discloses a process for the production of an oligosaccharide mixture derived from cow's milk comprising the steps a) concentrating a deproteinised cow's milk material to 50-75% total solids (TS); b) subjecting the concentrated milk material to a lactose removal step to produce a liquor having a lactose:oligosaccharide ratio of less than 100; c) optionally clarifying said liquor; d) treating the optionally clarified liquor with β-galactosidase to produce a liquor comprising β-galactooligosaccharides; e) optionally demineralising by, for example, passing the liquor through a weak cation column and, optionally, a mixed bed column and/or an an ion exchange column; f) carrying out a nanofiltration step, which may be carried out before or after the optional demineralisation step, and must be carried out after or at the same time as the treatment with β- galactosidase.

### BRIEF SUMMARY

The present disclosure describes processes and systems for preparing one or more galacto-oligosaccharide products ("GOS products") from one or more permeate byproducts, such as one or both of milk permeate or whey permeate. Examples of GOS products that may be prepared by the processes and systems described herein include, but are not limited to, a GOS syrup, a GOS gel, or a GOS powder (not part of the invention).

The resulting GOS products provided by the processes and systems described herein have composition profiles that are different from current commercially available GOS products, which results in digestive and other health benefits compared to known GOS products.

The invention relates to a process for producing a galacto-oligosaccharide product, the process comprising:exposing a permeate composition comprising a whey permeate, a milk permeate, or a mixture of a whey and milk permeate to one or more enzymes, wherein the permeate composition comprises a minimum lactose content of 76 wt%; wherein the one or more enzymes convert one or more compounds in the permeate composition to one or more galacto-oligosaccharides to provide a galacto-oligosaccharide solution, wherein at least 10% of total sugar, by weight, in the galacto-oligosaccharide solution is in the form of the one or more galacto-oligosaccharides;concentrating at least a portion of the galacto-oligosaccharide solution to provide a galacto-oligosaccharide syrup; and drying the galacto-oligosaccharide syrup to provide a flowable powder without the use of a drying agent compound.

In some examples, the present disclosure describes a galacto-oligosaccharide product composition (not part of the invention) comprising a dry or substantially dry powder including one or more dried compounds from a permeate composition and at least 20% by weight of one or more galacto-oligosaccharides,wherein the powder is free from a drying agent.

The invention is defined by the appended set of claims.

### BRIEF DESCRIPTION OF THE SEVERAL VIEWS OF THE DRAWINGS

**FIG. 1** is a flow diagram of an example process of preparing a galacto-oligosaccharide product from one or more permeate byproducts in the form of a galacto-oligosaccharide syrup or gel.
**FIG. 2** is a flow diagram of another example process of preparing a galacto-oligosaccharide product from one or more permeate byproducts in the form of a galacto-oligosaccharide powder.
**FIG. 3** is a flow diagram of another example process of preparing a galacto-oligosaccharide product from one or more permeate byproducts in the form of a low-salt galacto-oligosaccharide syrup or powder.
**FIG. 4** is a flow diagram of another example process of preparing a galacto-oligosaccharide product from one or more permeate byproducts in the form of a low-salt, low-glucose, and low-galactose galacto-oligosaccharide syrup or powder.
**FIG. 5** is a flow diagram of another example process of preparing a galacto-oligosaccharide product from one or more dried permeate byproducts.
**FIG. 6** is a profile graph of monosaccharides that resulted from HPAEC-PAD analysis of a galacto-oligosaccharide powder sample as produced in EXAMPLE 6.
**FIG. 7** is a profile graph of monosaccharides that resulted from HPAEC-PAD analysis of a galacto-oligosaccharide powder sample as produced in EXAMPLE 7.
**FIG. 8** is a HPAEC profile graph for various carbohydrate fractions in a standards solution including glucose, galactose, lactose, maltotriose, maltotetraose, and maltopentaose.
**FIG. 9** is a HPAEC profile graph for various carbohydrate fractions in the galacto-oligosaccharide powder sample produced in EXAMPLE 6.
**FIG. 10** is a HPAEC profile graph for various carbohydrate fractions in the galacto-oligosaccharide powder sample produced in EXAMPLE 7.
**FIG. 11** is a profile of a MALDI-TOF mass spectrometry analysis of the galacto-oligosaccharide powder sample produced in EXAMPLE 6.
**FIG. 12** is a profile of a MALDI-TOF mass spectrometry analysis of the galacto-oligosaccharide powder sample produced in EXAMPLE 7.

### DETAILED DESCRIPTION

The present invention relates to processes for producing galacto-oligosaccharide products from one or more milk or whey byproducts, such as one or more permeate byproducts, such as one or both of milk permeate or whey premeate. Also disclosed are the resulting galacto-oligosaccharide products (not part of the invention) from those processes. The galacto-oligosaccharide products can be used as food additives, either for human or animal consumption. The galacto-oligosaccharide products are unique compared to known commercial galacto-oligosaccharide products, and have unique health and digestive benefits compared to known commercial galacto-oligosaccharide products.

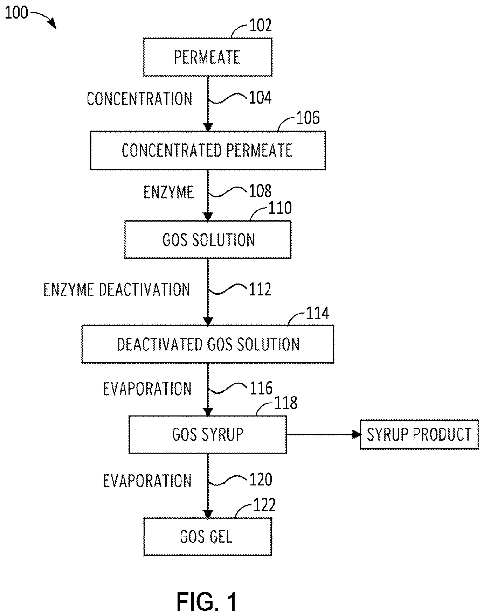

**FIG. 1** shows a flow diagram of an example process 100 for preparing a galacto-oligosaccharide (hereinafter "GOS") composition from a liquid dairy byproduct, such as one or more permeate byproducts 102. In an example, the one or more permeate byproducts 102 includes one or both of a whey permeate byproduct from a cheese manufacturing process (referred to hereinafter as simply "whey permeate") or a milk permeate byproduct from a milk protein manufacturing process (referred to hereinafter as simply "milk permeate"). For the sake of brevity, the one or more permeate byproducts 102 will simply be referred to as "permeate 102." However, a person of ordinary skill in the art will understand that when the term "permeate," as used herein, can refer to one or both of milk permeate or whey permeate, e.g., milk permeate, whey permeate, or a mixture of milk permeate and whey permeate. The same holds true for the feedstock permeate 102, and to other forms of permeate described below (e.g., a concentrated permeate after a concentration step comprises, in some examples, a concentrated milk permeate, a concentrated whey permeate, or a mixture of a concentrated milk permeate and a concentrated whey permeate). In an example, the permeate 102 consists essentially of, or consists of, a milk permeate, a whey permeate, or a mixture of a milk permeate and a whey permeate.

In an example, the permeate 102 has a solids content of about 5% to about 10%, by weight, total solids (hereinafter "TS"), such as from about 5 % to about 7% TS. In examples, the permeate 102 has a specified content of one or more components present in the permeate 102. In an example, the specified components of the permeate 102 include, but are not limiting to: protein, fats, sugars such as lactose, ash, moisture, sodium, calcium, magnesium, or potassium. As used herein, the term "protein" refers to a compound comprising one or more polypeptide chains (e.g., polymer chains of amino acid residues). As is typical with permeate byproducts, the "protein" that may be present in the permeate 102 can include several protein compounds.

As used herein, the term "fat" or "fats" refers to one or more triglyceride-based compounds that are sometimes also referred to as a "lipid" or "lipids."

As used herein, the term "sugar" or "sugars" refers to one or more short-chain carbohydrate compounds, and in particular to those comprising one saccharide group, also referred to as monosaccharides, and those comprising two saccharide groups, also referred to a disaccharides. A particularly common sugar present in dairy products is lactose. Other sugars may be present in small amounts, such as galactose or glucose.

As used herein, the term "ash" typically refers to a mineral rich fraction of the composition in question, and is sometimes simply referred to herein as "mineral" or "minerals." In some examples, ash can include, but is not limited to, compounds that include sodium (Na), potassium (K), calcium (Ca), magnesium (Mg), phosphorous (P), and chloride (CI).

As used herein, the term "moisture" refers to the portion of the composition that is made up of water (H₂O).

In particular, any of the components present in the permeate 102, or any other permeate described herein, that are described or defined herein include, but are not limited to, any component (e.g., protein, fats, lactose or other sugars, ash, moisture, sodium, calcium, magnesium, or potassium) that are typically present in dairy-based products, such a permeate byproduct, including the whey permeate or milk permeate products described above.

In an example, the permeate 102 has the content specified below for one or more of: protein, fats, lactose, ash, moisture, sodium, calcium magnesium, or potassium. In an example, the permeate 102 has the content specified below for all of protein, fats, lactose, ash, moisture, sodium, calcium magnesium, and potassium.

In an example, the permeate 102 can have a protein content of from about 1 wt.% to about 10 wt.%. In some examples, a whey permeate, which can be all of, or a portion of, the permeate 102, typically has a protein content of from about 2 wt.% to about 7 wt.%, with a typical maximum protein content of about 7 wt.%. In some examples, a milk permeate, which can be all of, or a portion of, the permeate 102, typically has a protein content of from about 3 wt.% to about 5 wt.%, with a typical minimum protein content of about 2 wt.%.

In an example, the permeate 102 can have a fat content of from about 0 wt.% to about 2 wt.%. In some examples, a whey permeate, which can be all of, or a portion of, the permeate 102, typically has a fat content of from about 0 wt.% to about 1 wt.%, with a typical maximum fat content of about 1.5 wt.%. In some examples, a milk permeate, which can be all of, or a portion of, the permeate 102, typically has a fat content of from about 0 wt.% to about 1 wt.%, with a typical maximum fat content of about 1.5 wt.%.

The whey permeate, which can be all of, or a portion of, the permeate 102, typically has a lactose content of from 76 wt.% to 85 wt.%, with a typical minimum lactose content of about 76 wt.%. In some examples, a milk permeate, which can be all of, or a portion of, the permeate 102, typically has a lactose content of from about 78 wt.% to about 88 wt.%, with a typical minimum lactose content of about 78 wt.%.

In an example, the permeate 102 can have an ash content of from about 5 wt.% to about 15 wt.%. In some examples, a whey permeate, which can be all of, or a portion of, the permeate 102, typically has an ash content of from about 8 wt.% to about 11 wt.%, with a typical maximum ash content of about 14 wt.%. In some examples, a milk permeate, which can be all of, or a portion of, the permeate 102, typically has an ash content of from about 8 wt.% to about 11 wt.%, with a typical maximum ash content of about 14 wt.%.

In an example, the permeate 102 can have a sodium content of from about 0.3 wt.% to about 1 wt.%. In some examples, a whey permeate, which can be all of, or a portion of, the permeate 102, typically has a sodium content of from about 0.7 wt.% to about 0.89 wt.%. In some examples, a milk permeate, which can be all of, or a portion of, the permeate 102, typically has a sodium content of from about 0.38 wt.% to about 0.66 wt.%.

In an example, the permeate 102 can have a calcium content of from about 0.3 wt.% to about 0.7 wt.%. In some examples, a whey permeate, which can be all of, or a portion of, the permeate 102, typically has a calcium content of from about 0.36 wt.% to about 0.62 wt.%. In some examples, a milk permeate, which can be all of, or a portion of, the permeate 102, typically has a calcium content of from about 0.36 wt.% to about 0.46 wt.%.

In an example, the permeate 102 can have a magnesium content of from about 0.05 wt.% to about 0.15 wt.%. In some examples, a whey permeate, which can be all of, or a portion of, the permeate 102, typically has a magnesium content of from about 0.1 wt.% to about 0.13 wt.%. In some examples, a milk permeate, which can be all of, or a portion of, the permeate 102, typically has a magnesium content of from about 0.1 wt.% to about 0.12 wt.%.

In an example, the permeate 102 can have a potassium content of from about 1.5 wt.% to about 5.5 wt.%. In some examples, a whey permeate, which can be all of, or a portion of, the permeate 102, typically has a potassium content of from about 2.2 wt.% to about 5.4 wt.%, such as from about 2.18 wt.% to about 5.36 wt.%. In some examples, a milk permeate, which can be all of, or a portion of, the permeate 102, typically has a potassium content of from about 1.9 wt.% to about 2.6 wt.%, such as from about 1.91 wt.% to about 2.58 wt.%.

In some examples, the permeate 102 is concentrated in a concentration operation 104 to provide a concentrated permeate 106 (e.g., one or both of a concentrated milk permeate or a concentrated whey permeate). In some examples, the concentration 104 of the permeate 102 is performed via one or both of reverse osmosis or evaporation. In examples where the concentration 104 comprises reverse osmosis, the concentration 104 includes a reverse osmosis process that concentrates the permeate 102. As used herein, in some examples, "reverse osmosis" refers to any process involving concentration of a process stream via the use of reverse osmosis membrane filtration technology. For the purposes of brevity, reverse osmosis will be referred to herein as "RO." Concentration 104 via RO can include the use of any RO membrane filtration technology capable of concentrating the permeate 102 to a desired solids content for the concentrated permeate 106. Examples of RO membrane filtration technology used for the concentration 104 include, but are not limited to, at least one of: one or more semi permeable membranes, one or more thin-film composite membranes, one or more spiral wound membranes, one or more thin sheet membranes, one or more hollow fiber membranes, one or more cellulose acetate membranes, or one or more polyamide membranes.

In examples where the concentration 104 comprises evaporation, the concentration 104 includes concentrating the permeate 102 in an evaporator, for example to provide a desired solids concentration of the concentrated permeate 106. Examples of evaporators used in the concentration 104 include, but are not limited to, a rising film evaporator, a falling film evaporator, a single effect evaporator, a multiple effect evaporator, a flash evaporator, a vacuum evaporator, a centrifugal evaporator, a rotary evaporator, or a swept surface evaporator.

As shown in the process 100 of **FIG. 1****,** the permeate 102 is subjected to concentration 104, for example by one or both of RO and evaporation, to provide the concentrated permeate 106. In some examples, the concentrated permeate 106 has a solids content of from about 20% to about 50% TS, such as from about 30% to about 40% TS. In some examples, the specific concentration of the concentrated permeate 106 is selected to provide for specified characteristics of the resulting final product, e.g., to provide for specified compositions or physical characteristics, or both, of the resulting syrup, gel, powder, or other form of GOS composition that results from the process.

After providing the concentrated permeate 106 with the desired solids concentration from the permeate 102, the concentrated permeate 106 is subjected to an enzyme treatment 108 that converts at least a portion of the concentrated permeate 106 to a solution 110 comprising one or more galacto-oligosaccharide compounds, referred to herein as the "GOS solution 110" for the sake of brevity. In an example, from about 10% to about 50% of the total sugar in the GOS solution 110 after the enzyme treatment 108 is in the form of one or more galacto-oligosaccharides (GOS), such as about 20% to about 30% of the sugars in the GOS solution 110.

The enzyme treatment 108 includes exposing the concentrated permeate 106 to one or more enzymes that convert one or more compounds in the concentrated permeate 106 to one or more GOS compounds. In an example, the one or more enzymes comprise a β-galactosidase, such as at least one of a β-galactosidase from the fungus *Aspergillus oryzae* or a β-galactosidase from the yeast *Kluveromyces lactis.* In an example, the enzyme treatment 108 comprises exposing the concentrated permeate 106 to only the β-galactosidase from *Aspergillus oryzae.* In an example, the enzyme treatment 108 comprises exposing the concentrated permeate 106 to only the β-galactosidase from *Kluveromyces lactis.* In an example, the enzyme treatment 108 comprises exposing the concentrated permeate 106 to an enzyme combination comprising β-galactosidase from *Aspergillus oryzae* and β-gaiactosidase from *Kluveromyces lactis.*

In some examples, the concentration of the one or more enzymes used in the enzyme treatment 108 is from about 0.001% to about 0.2%, by weight, such as from about 0.01% to about 0.05% by weight. In some examples, the specific relative composition of the one or more enzymes for the enzyme treatment 108, or the concentration of the one or more enzymes used in the enzyme treatment 108, or both is selected to provide for specified characteristics of the resulting final product, e.g., to provide for specified compositions or physical characteristics, or both, of the resulting syrup, gel, powder, or other form of GOS composition that results from the process. For example, if the enzyme treatment 108 is performed with a combination of the β-galactosidases from *Aspergillus oryzae* and *Kluveromyces lactis,* then the relative concentration of each enzyme in the mixture is modified to achieve different characteristics of the resulting galacto-oligosaccharide syrup 118 or galacto-oligosaccharide gel 122 (described in more detail below).

In some examples, the one or more enzymes are hydrated in a solution of potable process water prior to the enzyme treatment 108. In some examples, the temperature at which the one or more enzymes is hydrated is from about 60 °F (about 15.6°C) to about 90°F (about 32.2 °C), such as from about 70 °F (about 21.1°C) to about 80 °F (about 26.7 °C).

In some examples, during the enzyme treatment 108 the one or more enzymes are allowed to react with the concentrated permeate 106 for a reaction time of from about 1 hour to about 8 hours, such as from about 2 hours to about 4 hours. In some examples, the temperature at which the enzyme treatment 108 is performed, e.g., the temperature at which the concentrated permeate 106 is exposed to the one or more enzymes, is from about 120 °F (about 48.9 °C) to about 150 °F (about 65.6 °C), such as from about 125 °F (about 51.7 °C) to about 135 °F (about 57.2 °C). In some examples, the specific temperature that the enzyme treatment 108 is performed at is selected to provide for specified characteristics of the resulting final product, e.g., to provide for specified compositions or physical characteristics, or both, of the resulting syrup, gel, powder, or other form of GOS composition that results from the process.

In some examples, after the enzyme treatment 108 that provides the GOS solution 110, the GOS solution 110 is subjected to an enzyme deactivation 112 to deactivate the one or more enzymes from the enzyme treatment 108. The enzyme deactivation 112 also inhibits conversion of a least a portion of the concentrated permeate 106 to a GOS solution 110, e.g., by stopping the conversion of one or more compounds in the concentrated permeate 106 to GOS. In an example, the enzyme deactivation 112 is via a heat treatment process by which the temperature of the GOS solution 110 is raised to a temperature greater than or equal to about 150 °F (about 65.6 °C). The enzyme deactivation 112 provides a deactivated GOS solution 114.

In some examples, after the enzyme treatment 108 that provides the GOS solution 110, the deactivated GOS solution 114 is further concentrated, for example via evaporation 116. In some examples, the evaporation 116 also is under conditions that are sufficient to deactivate the one or more enzymes used in the enzyme treatment 108 if they were not deactivated during the operation of enzyme deactivation 112 described above. In other words, in some examples, the enzyme deactivation 112 and the evaporation 116 are preformed in the same or substantially the same process step.

The evaporation 116 of the deactivated GOS solution 114 concentrates the deactivated GOS solution 114 to provide a galacto-oligosaccharide syrup 118 (herein referred to as a "GOS syrup 118"). In an example, the evaporation 116 concentrates the deactivated GOS solution 114 to a solids content of from about 50% to about 75% TS, such as from about 60% to about 70% TS. The evaporation 116 is performed in any type of evaporator that is capable of concentrating the deactivated GOS solution 114 to the desired solids concentration of the GOS syrup 118. Examples of types of evaporators that are used in the evaporation 116 to form the GOS syrup 118 include, but are not limited to, a rising film evaporator, a falling film evaporator, a single effect evaporator, a multiple effect evaporator, a flash evaporator, a vacuum evaporator, a centrifugal evaporator, a rotary evaporator, or a swept surface evaporator.

In an example, the GOS syrup 118 is the final product of the process 100, e.g., as indicated by the "syrup produce 119" process stream shown in FIG. 1. In other examples, the GOS syrup 118 is further concentrated via evaporation 120 to provide a galacto-oligosaccharide gel 122 (referred to herein as a "GOS gel 122"). In an example, the GOS gel 122 has a solids content of from about 75% to about 85% TS, such as from about 75% to about 80% TS. The evaporation 120 is performed in any type of evaporator that is capable of concentrating the GOS syrup 118 to the desired solids concentration of the GOS gel 122. Examples of types of evaporators that are used in the evaporation 120 to form the GOS gel 122 include, but are not limited to, a rising film evaporator, a falling film evaporator, a single effect evaporator, a multiple effect evaporator, a flash evaporator, a vacuum evaporator, a centrifugal evaporator, a rotary evaporator, or a swept surface evaporator.

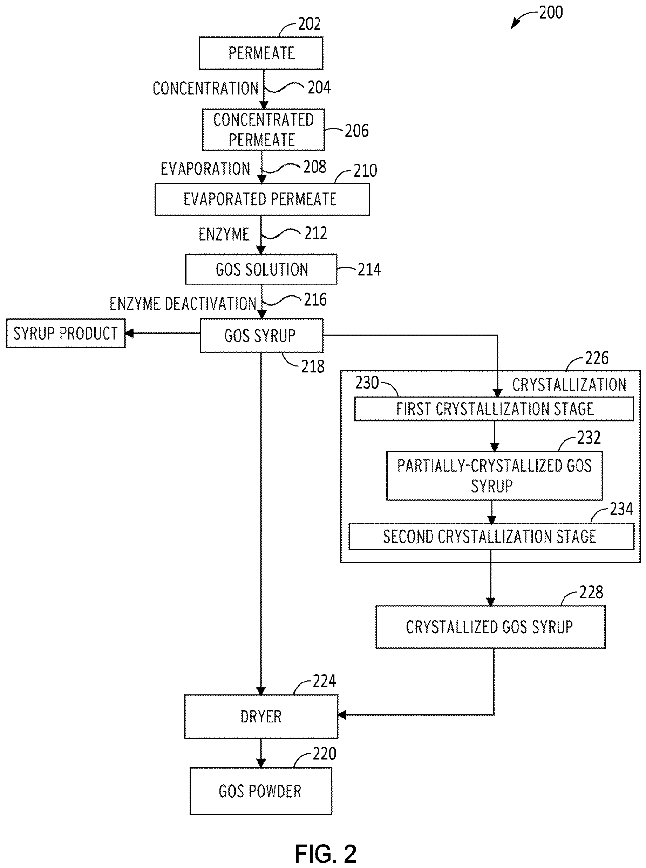

**FIG. 2** shows a flow diagram of another example process 200 for preparing a GOS product from a dairy byproduct, such as one or more permeate byproducts 202, for example at least one of a milk permeate or a whey permeate. As with the one or more permeate byproducts 102 described above in the process 100 of **FIG. 1****,** the one or more permeate byproducts 202 will be referred to simply as "permeate 202" for brevity. In an example, the permeate 202 comprises a milk permeate, a whey permeate, or a mixture of a milk permeate and a whey permeate. In an example, the permeate 202 consists essentially of, or consists of, a milk permeate, a whey permeate, or a mixture of a milk permeate and a whey permeate.

The permeate 202 that is the feedstock for the process 200 can be similar to the permeate 102 described above for the process 100. In an example, the permeate 202 can be identical or substantial identical to the permeate 102 described above. In an example, the permeate 202 has the same solids content as specified above with respect to the permeate 102. In an example, the permeate 202 has the same specified content of one or more components present in the permeate 202 as described above with respect to the permeate 102, e.g., the specified content of one or more of, and in some examples all of: protein, fats, lactose, ash, moisture, sodium, calcium magnesium, or potassium.

The process 200 of **FIG. 2** can be thought of as an alternative to that of process 100, with the process 200 producing a GOS product in another form from the GOS syrup 118 or the GOS gel 122 that result from the process 100. Like the permeate 102 in process 100, in some examples, the permeate 202 is concentrated in a concentration operation 204 to provide a concentrated permeate 206 (e.g., one or both of a concentrated milk permeate or a concentrated whey permeate). In some examples, the concentration 204 of the permeate 202 is substantially similar or identical to the concentration 104 described above. In an example, the concentration 204 of the permeate 202 includes one or both of reverse osmosis ("RO") or evaporation. In examples where the concentration 204 comprises RO, the concentration 204 includes concentrating of the permeate 202 via the use of RO membrane filtration technology. Concentration 204 via RO can include the use of any RO membrane filtration technology capable of concentrating the permeate 202 to a desired solids content for the concentrated permeate 206. Examples of RO membrane filtration technology used for the concentration 204 include, but are not limited to, at least one of: one or more semi permeable membranes, one or more thin-film composite membranes, one or more spiral wound membranes, one or more thin sheet membranes, one or more hollow fiber membranes, one or more cellulose acetate membranes, or one or more polyamide membranes.

In examples where the concentration 204 comprises evaporation, the concentration 204 includes concentrating the permeate 202 in an evaporator. The evaporator used for the concentration 204 can be any type of evaporator capable of concentrating the permeate 202 to a desired solids concentration for the concentrated permeate 206. Examples of evaporators used in the concentration 204 include, but are not limited to, a rising film evaporator, a falling film evaporator, a single effect evaporator, a multiple effect evaporator, a flash evaporator, a vacuum evaporator, a centrifugal evaporator, a rotary evaporator, or a swept surface evaporator. In an example, after the concentration step 204, the resulting concentrated permeate 206 can have a solids content of from about 20% to about 50% TS, such as from about 30% to about 40% TS.

In some examples, after the concentration 204, the concentrated permeate 206 is further concentrated, such as via an evaporation operation 208 to provide an evaporated permeate 210. In an example, the evaporated permeate 210 has a solids content of from about 50% to about 80% TS, such as from about 60% to about 70% TS. In some examples, the specific concentration of the evaporated permeate 210 is selected to provide for specified characteristics of the resulting final product, e.g., to provide for specified compositions or physical characteristics, or both, of the resulting syrup, gel, powder, or other form of GOS composition that results from the process.

In an example, the evaporation 208 is performed in any type of evaporator that is capable of concentrating the concentrated permeate 206 to the desired solids concentration for the evaporated permeate 210. Examples of evaporators that can be used for the evaporation 208 include, but are not limited to, a rising film evaporator, a falling film evaporator, a single effect evaporator, a multiple effect evaporator, a flash evaporator, a vacuum evaporator, a centrifugal evaporator, a rotary evaporator, or a swept surface evaporator.

Next, the evaporated permeate 210 is subjected to an enzyme treatment 212 to convert at least a portion of the evaporated permeate 210 to a solution 214 including one or more galacto-oligosaccharide compounds, referred to herein as the "GOS solution 214" for the sake of brevity. In some examples, the enzyme treatment 212 is substantially similar to the enzyme treatment 108, except that it is being performed on the evaporated permeate 210, which has a higher solids content than the concentrated permeate 106 in the process 100. In an example, from about 10% to about 50% of the total sugar in the GOS solution 214 after the enzyme treatment 212 is in the form of one or more galacto-oligosaccharides (GOS), such as from about 20% to about 30% of the sugars in the GOS solution 214.

In an example, the enzyme treatment 212 comprises exposing the evaporated permeate 210 to one or more enzymes capable of converting one or more compounds in the evaporated permeate 210 to one or more GOS compounds, similar to the enzyme treatment 108. In an example, the one or more enzymes comprise one or more β-galactosidase enzymes, such as one or both of the β-galactosidase from *Aspergillus oryzae* and the β-galactosidase from *Kluveromyces lactis.* In an example, the enzyme treatment 212 comprises exposing the evaporated permeate 210 to only the β-galactosidase from *Aspergillus oryzae.* In an example, the enzyme treatment 212 comprises exposing the evaporated permeate 210 to only the β-galactosidase from *Kluveromyces lactis.* In an example, the enzyme treatment 212 comprises exposing the evaporated permeate 210 to an enzyme combination comprising β-galactosidase from *Aspergillus oryzae* and β-galactosidase from *Kluveromyces lactis.*

In an example, the concentration of the one or more enzymes used in the enzyme treatment 212 is from about 0.001% to about 0.2%, by weight, such as from about 0.01% to about 0.05% by weight. In an example, the one or more enzymes is hydrated in a solution of potable process water prior to the enzyme treatment 212. In some examples, the specific relative composition of the one or more enzymes for the enzyme treatment 212, or the concentration of the one or more enzymes used in the enzyme treatment 212, or both is selected to provide for specified characteristics of the resulting final product, e.g., to provide for specified compositions or physical characteristics, or both, of the resulting syrup, gel, powder, or other form of GOS composition that results from the process. For example, if the enzyme treatment 212 is performed with a combination of the β-galactosidases from *Aspergillus oryzae* and *Kluveromyces lactis,* then the relative concentration of each enzyme in the mixture is modified to achieve different characteristics of the resulting galacto-oligosaccharide syrup 218 or galacto-oligosaccharide powder 220 (described in more detail below).

In an example, the temperature at which the one or more enzymes is hydrated is from about 60 °F (about 15.6 °C) to about 90 °F (about 32.2 °C), such as from about 70 °F (about 21.1 °C) to about 80 °F (about 26.7 °C).

As with the enzyme treatment 108 that provides the GOS solution 214, in an example, during the enzyme treatment 212, the one or more enzymes are allowed to react with the evaporated permeate 210 for a reaction time of from about 1 hour to about 8 hours, such as from about 2 hours to about 4 hours. In an example, the temperature at which the enzyme treatment 212 is performed, e.g., the temperature at which the evaporated permeate 210 is exposed to the one or more enzymes, is from about 120 °F (about 48.9 °C) to about 150 °F (about 65.6 °C), such as from about 125 °F (about 51.7 °C) to about 135 °F (about 57.2 °C). In some examples, the specific temperature that the enzyme treatment 212 is performed at is selected to provide for specified characteristics of the resulting final product, e.g., to provide for specified compositions or physical characteristics, or both, of the resulting syrup, gel, powder, or other form of GOS composition that results from the process.

In some examples, after the enzyme treatment 212 that provides the GOS solution 214, the GOS solution 214 is subjected to an enzyme deactivation 216 to deactivate the one or more enzymes from the enzyme treatment 212. In an example, the enzyme deactivation 216 inhibits conversion of at least a portion of the compounds from the evaporated permeate 210 to GOS in the GOS solution 214. In an example, the enzyme deactivation 216 comprises heating the GOS solution 214 to a temperature that at least partially deactivates the one or more enzymes from the enzyme treatment 212. In an example, the enzyme deactivation 216, in addition to deactivating the one or more enzymes from the enzyme treatment 212, includes concentrating the GOS solution 214 by evaporation to provide a galacto-oligosaccharide syrup 218 (hereinafter referred to as a "GOS syrup 218"). In an example, the evaporation is performed under conditions that are sufficient to deactivate the one or more enzymes used in the enzyme treatment 212. In an example, the enzyme deactivation 216 comprises any heat treatment process by which the temperature of the GOS solution 214 is raised to greater than or equal to 150 °F (about 65.6 °C) to provide the GOS syrup 218.

In an example, the GOS syrup 218 is the final product of the process 200, e.g., as indicated by the "syrup produce 219" process stream shown in FIG. 2. In other examples, the process 200 includes further processing of the GOS syrup 218, e.g., to provide other forms of GOS products. In an example, the process 200 includes drying the GOS syrup 218 to provide a solid or substantially solid galacto-oligosaccharide product, such as a galacto-oligosaccharide powder 220 (hereinafter "GOS powder 220"). In an example, the process 200 includes sending at least a portion of the GOS syrup 218 to a dryer 224 capable of drying the GOS syrup 218 to a moisture content level that results in the formation of the solid or substantially solid GOS product desired, e.g., the GOS powder 220. Examples of dryers 224 used to for drying the GOS syrup 218 to form the GOS powder 220 include, but are not limited to, one or more of a pulse combustion dryer, a fluid bed dryer, a rotary dryer, a spray dryer, a roller dryer, a vacuum dryer, a box dryer, a cyclone dryer, a drum dryer, or a baghouse dryer.

In an example, the process 200 includes feeding at least a portion of the GOS syrup 218 directly into the dryer 224. In another example, the process 200 includes subjecting at least a portion of the GOS syrup 218 to a crystallization operation 226 to provide a crystallized GOS syrup 228. In an example, at least a portion of the crystallized GOS syrup 228 is fed into the dryer 224 to produce the GOS powder 220. In some examples, the crystallization 226 crystallizes one or more of, and in some examples all three of: at least a portion of the lactose in the GOS syrup 218; at least a portion of lactose-hydrolyzed components in the GOS syrup 218; or at least a portion of one or more GOS compounds in the GOS syrup 218.

In an example, the crystallization 226 comprises a two-stage process. In an example, the crystallization 226 includes a first crystallization stage 230 performed at a first temperature. In an example, the first temperature of the first crystallization stage 230 at least partially crystallizes at least a portion of the lactose in the GOS syrup 218 to provide a partially-crystallized GOS syrup 232. As used herein, the term "partially crystallized" when referring to the partially-crystallized GOS syrup 232, refers to at least about 5 wt% of the lactose present in the GOS syrup 218 is in a crystallized solid form in the partially-crystallized GOS syrup 232, such as at least about 10 wt%, at least about 15 wt.%, at least about 20 wt.%, or at least about 25 wt.% of the lactose in the GOS syrup 218 having been crystallized. In some examples, the GOS syrup 232 will be considered "at least partially crystallized" when at least about 5 wt.% of the lactose-hydrolyzed components originally present in the GOS syrup 218 are in a crystallized solid form, such as at least about 10 wt%, at least about 15 wt.%, at least about 20 wt.%, or at least about 25 wt.% of the lactose-hydrolyzed components having been crystallized, in the partially-crystallized GOS syrup 232. In some examples, the partially-crystallized GOS syrup 232 will considered "partially crystallized" when at least about 5 wt.% of the galacto-oligosaccharide compounds present in the GOS syrup 218 are in a crystallized solid form in the partially-crystallized GOS syrup 232, such as at least about 10 wt%, at least about 15 wt.%, at least about 20 wt.%, or at least about 25 wt.% of the galacto-oligosaccharide compounds present in the GOS syrup 218 having been crystallized in the partially-crystallized GOS syrup 232. In some examples, the partially-crystallized GOS syrup 232 will be considered "partially crystallized" when at least about 5 wt.% of all three of the lactose, the lactose-hydrolyzed components, and the galacto-oligosaccharide compounds present in the GOS syrup 218 are in a crystallized solid form in the partially-crystallized GOS syrup 232, such as at least about 10 wt%, at least about 15 wt.%, at least about 20 wt.%, or at least about 25 wt.% of the lactose, the lactose-hydrolyzed components, and the galacto-oligosaccharide compounds present in the GOS syrup 218 having been crystallized in the partially-crystallized GOS syrup 232. In an example, the first temperature of the first crystallization stage 230 is from about 60 °F (about 15 °C or about 16 °C) to about 100 °F (e.g., about 37 °C or about about 38 °C), such as from about 80 °F (e.g., about 25 °C to about 27 °C) to about 95 °F (e.g., about 35 °C).

In an example, the partially crystallized GOS syrup 232 is subjected to a second crystallization stage 234 performed at a second temperature. In an example, the second temperature of the second crystallization stage 234 is lower than the first temperature of the first crystallization stage 230. In an example, the second crystallization stage 234 provides a fully or substantially fully crystallized GOS syrup 228. In an example, the phrase "fully or substantially fully crystallized," as used with reference to the crystallized GOS syrup 228, refers to the crystallization of one or more of the lactose, the one or more lactose-hydrolyzed components, or the one or more galacto-oligosaccharide compounds in the crystallized GOS syrup 228. In some examples, the GOS syrup 228 will be considered "substantially fully crystallized" when at least about 50 wt.% of the lactose present in the GOS syrup 218 is in a crystallized solid form in the crystallized GOS syrup 228, such as at least about 55 wt.%, at least about 60 wt.%, at least about 65 wt.%, at least about 70 wt.%, at least about 75 wt.%, at least about 80 wt.%, at least about 85 wt.%, at least about 90 wt.%, or at least about 95 wt.% of the lactose originally present in the GOS syrup 218 having been crystallized in the crystallized GOS syrup 228. In some examples, the GOS syrup 228 will be considered "substantially fully crystallized" when at least about 50 wt.% of the lactose-hydrolyzed components present in the GOS syrup 218 are in a crystallized solid form in the crystallized GOS syrup 228, such as at least about 55 wt.%, at least about 60 wt.%, at least about 65 wt.%, at least about 70 wt.%, at least about 75 wt.%, at least about 80 wt.%, at least about 85 wt.%, at least about 90 wt.%, or at least about 95 wt.% of the lactose-hydrolyzed components originally present in the GOS syrup 218 having been crystallized in the crystallized GOS syrup 228. In some examples, the crystallized GOS syrup 228 will considered "substantially fully crystallized" when at least about 50 wt.% of the galacto-oligosaccharide compounds present in the GOS syrup 218 are in a crystallized solid form in the crystallized GOS syrup 228, such as at least about 55 wt.%, at least about 60 wt.%, at least about 65 wt.%, at least about 70 wt.%, at least about 75 wt.%, at least about 80 wt.%, at least about 85 wt.%, at least about 90 wt.%, or at least about 95 wt.% of the galacto-oligosaccharide compounds in the GOS syrup 218 having been crystallized in the crystallized GOS syrup 228. In some examples, the GOS syrup 228 will be considered "substantially fully crystallized" when at least about 50 wt.% of all three of the lactose, the lactose-hydrolyzed components, and the galacto-oligosaccharide compounds present in the GOS syrup 218 are in a crystallized solid form in the crystallized GOS syrup 228, such as at least about 55 wt.%, at least about 60 wt.%, at least about 65 wt.%, at least about 70 wt.%, at least about 75 wt.%, at least about 80 wt.%, at least about 85 wt.%, at least about 90 wt.%, or at least about 95 wt.% of the lactose, the lactose-hydrolyzed components, and the galacto-oligosaccharide compounds from the GOS syrup 218 having been crystallized in the crystallized GOS syrup 228. In some examples, the crystallized GOS syrup 228 can include other crystallizable compounds that are uncrystallized or partially crystallized and the crystallized GOS syrup 228 will still be considered fully or substantially fully crystallized for the purposes of the process 200. In an example, the second temperature of the second crystallization stage 234 is from about 40 °F (e.g., about 4 °C or about 5 °C) to about 80 °F (e.g., about 25 °C to about 27 °C), such as from about 45 °F (e.g., about 7 °C) to about 60 °F (e.g., about 15 °C or about 16 °C).

In an example, the total time of the crystallization 226 (e.g., for both the first crystallization stage 230 and the second crystallization stage 234 combined) is from about 1 hour to about 10 hours. In some examples, the first crystallization stage 230 and the second crystallization stage 234 each take about the same length of time. After the crystallization 226, at least a portion of the crystallized GOS syrup 228 is fed to the dryer 224 to provide the GOS powder 220.

The GOS syrup 218 or the crystallized GOS syrup 228 prepared by the process 200 is such that drying with just the dryer 224 (in the case of the GOS syrup 218) or with the dryer 224 after a crystallization operation, such as the crystallization 226 (in the case of the crystallized GOS syrup 228) is dried to sufficiently low moisture to provide for a powdered product, such as a flowable powder, without the use of a drying agent.

In some examples, the GOS powder 220 comprises at least about 20 wt% of one or more galacto-oligosaccharide compounds, such as at least about 25 wt.%, at least about 30 wt.%, at least about 35 wt.%, at least about 40 wt%, at least about 45 wt%, or at least about 50 wt.% of one or more galacto-oligosaccharide compounds. In an example, the GOS powder 220 is from about 20 wt.% to about 40 wt.% galacto-oligosaccharide compounds, such as from about 20 wt.% to about 30 wt.% galacto-oligosaccharide. In some examples, the GOS powder 220 is from about 10 wt.% to about 25 wt.% glucose, such as from about 15 wt.% to about 20 wt.% glucose. In some examples, the GOS powder 220 is from about 2 wt.% to about 15 wt.% galactose, such as from about 5 wt.% to about 10 wt.% galactose. In some examples, the GOS powder 220 is from about 20 wt.% to about 35 wt.% lactose, such as from about 25 wt.% to about 30 wt.% lactose. In some examples, the GOS powder 220 is from about 1 wt.% to about 7 wt.% protein, such as from about 2 wt.% to about 5 wt.% protein. In some examples, the GOS powder 220 is from about 0 wt.% to about 1.5 wt.% fat, for example from about 0 wt.% to about 1 wt.% fat. In some examples, the GOS powder 220 is from about 2 wt.% to about 14 wt.% ash, such as from about 5 wt.% to about 10 wt.% ash. In some examples, the GOS powder 220 is from about 2 wt.% to about 5 wt.% moisture, such as about 3 wt.% moisture. In some examples, the GOS powder 220 is from about 0.4 wt.% to about 0.6 wt.% calcium. In some examples, the GOS powder 220 is from about 0.1 wt.% to about 0.15 wt.% magnesium. In some examples, the GOS powder 220 is from about 0.6 wt.% to about 0.8 wt.% phosphorous. In some examples, the GOS powder 220 is from about 2 wt.% to about 5 wt.% potassium. In some examples, the GOS powder 220 is from about 0.2 wt.% to about 1 wt.% sodium, such as from about 0.4 wt.% to about 0.9 wt.% sodium. In some examples, the GOS powder 220 is from about 0.5 wt.% to about 1.8 chloride, such as from about 0.6 wt.% to about 1 wt.% chloride. In some examples, the GOS powder 220 is from about 1 wt.% to about 4 wt.% salt, such as from about 2 wt.% to about 3 wt.% salt.

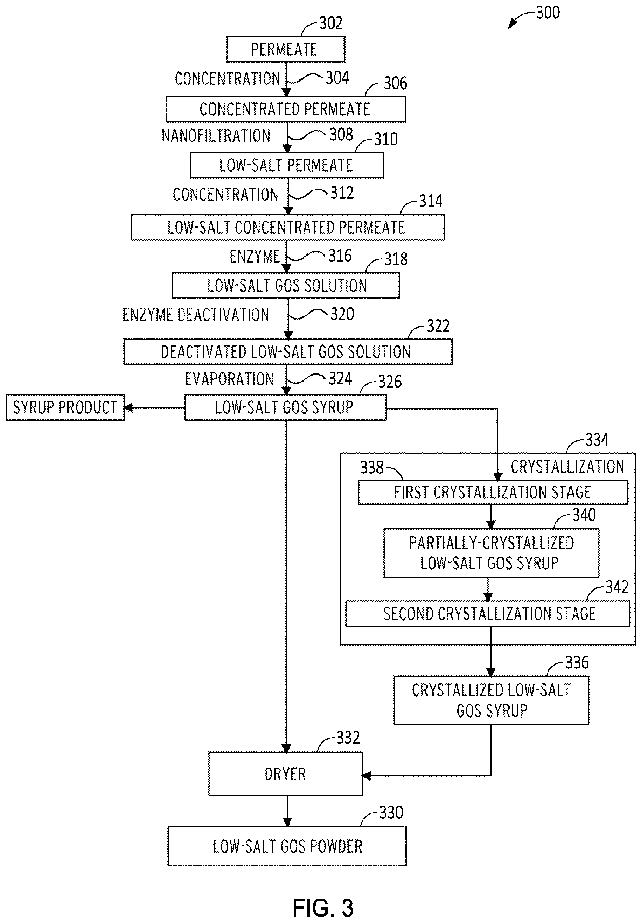

**FIG. 3** shows a flow diagram of another example process 300 for preparing a GOS product from a dairy byproduct, such as one or more permeate byproducts 302, for example at least one of a milk permeate or a whey permeate. As with the one or more permeate byproducts 102 and 202 described above, the one or more permeate byproducts 302 will be referred to simply as "permeate 302" for brevity. In an example, the permeate 302 comprises a milk permeate, a whey permeate, or a mixture of a milk permeate and a whey permeate. In an example, the permeate 302 consists essentially of, or consists of, a milk permeate, a whey permeate, or a mixture of a milk permeate and a whey permeate.

The permeate 302 that is the feedstock for the process 300 can be similar to the permeate 102 described above. In an example, the permeate 302 can be identical or substantial identical to the permeate 102 described above. In an example, the permeate 302 has the same solids content as specified above with respect to the permeate 102. In an example, the permeate 302 has the same specified content of one or more components present in the permeate 302 as described above with respect to the permeate 102, e.g., the specified content of one or more of, and in some examples all of: protein, fats, lactose, ash, moisture, sodium, calcium magnesium, or potassium.

The process 300 of **FIG. 3** can be thought of as an alternative to that of process 100 (**FIG. 1**) and process 200 (**FIG. 2**), with the process 300 producing a modified or enhanced form of the GOS products 118, 122, 218, and 222 described with respect to processes 100 and 200. The process 300 provides a GOS product with a reduced level of monovalent salts, such as sodium chloride (NaCl) or potassium chloride (KCl), compared to that of the GOS products 118, 122, 218, and 222.

In an example, the permeate 302 is concentrated in a concentration operation 304 to provide a concentrated permeate 306 (e.g., one or both of a concentrated milk permeate or a concentrated whey permeate). In some examples, the concentration 304 of the permeate 302 is substantially similar or identical to the concentration 104 and the concentration 204 described above. In an example, the concentration 304 of the permeate 302 includes one or both of reverse osmosis ("RO") or evaporation. In examples where the concentration 304 comprises RO, the concentration 304 includes concentrating the permeate 302 via the use of RO membrane filtration technology. Concentration 304 via RO can include the use of any RO membrane filtration technology capable to concentrating the permeate 302 to a desired solids content for the concentrated permeate 306. Examples of RO membrane filtration technology used for the concentration 304 include, but are not limited to, at least one of: one or more semi permeable membranes, one or more thin-film composite membranes, one or more spiral wound membranes, one or more thin sheet membranes, one or more hollow fiber membranes, one or more cellulose acetate membranes, or one or more polyamide membranes.

In examples where the concentration 304 comprises evaporation, the concentration 304 includes concentrating the permeate 302 in an evaporator. The evaporator used for the concentration 304 can be any type of evaporator capable of concentrating the permeate 302 to a desired solids content for the concentrated permeate 306. Examples of evaporators used in the concentration 304 include, but are not limited to, a rising film evaporator, a falling film evaporator, a single effect evaporator, a multiple effect evaporator, a flash evaporator, a vacuum evaporator, a centrifugal evaporator, a rotary evaporator, or a swept surface evaporator. In an example, after the concentration step 304, the resulting concentrated permeate 306 has a solids content of from about 10% to about 30% TS, such as from about 15% to about 25% TS.

In some examples, after the concentration 304, the concentrated permeate 306 is further processed to remove monovalent salt compounds from the concentrated permeate 306 to provide a low-salt permeate 310. In an example, shown in **FIG. 3****,** the concentrated permeate 306 is subjected to nanofiltration 308 (hereinafter "NF 308"), e.g., by being passed through a nanofiltration membrane, to remove monovalent salts from the concentrated permeate 306 in order to provide the low-salt permeate 310. Examples of monovalent salts that are removed from the concentrated permeate 306 by the NF 308 include, but are not limited to, one or more of sodium chloride (NaCl) or potassium chloride (KCl).

In an example, the NF 308 reduces monovalent salts in the concentrated permeate 306, on a dry weight basis, by at least about 50%, such as at least about 60%, for example at least about 75%, such as at least about 80%, and in some examples by about 89% or 90% or more. In an example, shown in the example of Table 2 (described in more detail below), the NF 308 reduces the dry-basis salt content by about 89%, from about 2.8 g/100 g dry product to about 0.3 g/100g in the low-salt permeate 310. In an example, shown in Table 3 (described in more detail below), the NF 308 results in a reduction of sodium, on a dry weight basis, of about 80%, from about 0.7 g/100 g dry product to about 0.14 g/100 g for the low-salt permeate 310. In an example, the NF 308 results in a reduction of other components from the concentrated permeate 306, such as other minerals (e.g., potassium, chloride, and to a lesser extent, iron, zinc, and phosphorus) and non-protein nitrogen (NPN).

In some examples, the specific target composition of the low-salt permeate 310 is selected to provide for specified characteristics of the resulting final product, e.g., to provide for specified compositions or physical characteristics, or both, of the resulting syrup, gel, powder, or other form of GOS composition that results from the process.

The nanofiltration membrane used for the NF 308 provides for the selective rejection of salts from the concentrated permeate 306. The NF 308 is performed using any type of nanofiltration ("NF") process that is capable of achieving desired levels of salt, sodium, and other minerals, such as the levels described above, to provide the low-salt permeate 310 with a specified compositional profile, for example by utilizing NF membrane filtration technology. Examples of NF membrane filtration technology used for the NF 308 include, but are not limited to, semi-permeable membranes, thin-film composite membranes, spiral-wound membranes, thin-sheet membranes, hollow-fiber membranes, cellulose acetate membranes, or polyamide membranes.

**FIG. 3** shows the concentration 304 and the NF 308 being separate steps in the process 300, e.g., separate processing steps with separate processing equipment. However, the process 300 is not so limited, and a person of ordinary skill in the art will appreciate that processing the permeate 302 by concentration 304 and NF 308 can be combined or substantially combined into a single, or what is essentially a single, processing operation utilizing a single, or what is essentially a single, piece of equipment. For example, the permeate 302 can be processed by a reverse osmosis/nanofiltration process, referred to herein as "RO/NF" or an "RO/NF process." In examples where the concentration 304 and NF 308 are combined in an RO/NF process, the RO/NF process is performed using any type of RO and NF technology that is capable of providing the desired compositional profile for the low-salt permeate 310, for example with the monovalent salt, sodium, and other minerals profile described above, and that utilizes RO and NF membrane filtration technology. Examples of RO and NF membrane filtration technology used in a RO/NF process include, but are not limited to, semi permeable membranes, thin-film composite membranes, spiral wound membranes, thin sheet membranes, hollow fiber membranes, cellulose acetate membranes, or polyamide membranes.

In some examples, after the concentration and NF of the permeate 302 (either as separate concentration 304 and NF 308 steps or as a combined RO/NF process), the low-salt permeate 310 is further concentrated via a concentration operation 312 to provide a low-salt concentrated permeate 314. In an example, the concentration 312 of the low-salt permeate 310 includes one or both of reverse osmosis ("RO") or evaporation. In examples where the concentration 312 comprises RO, the concentration 312 includes concentrating the low-salt permeate 310 via the use of RO membrane filtration technology. Concentration 312 via RO can include the use of any RO membrane filtration technology capable of concentrating the low-salt permeate 310 to a desired solids content for the low-salt concentrated permeate 314. Examples of RO membrane filtration technology used for the concentration 312 include, but are not limited to, at least one of: one or more semi permeable membranes, one or more thin-film composite membranes, one or more spiral wound membranes, one or more thin sheet membranes, one or more hollow fiber membranes, one or more cellulose acetate membranes, or one or more polyamide membranes.

In examples where the concentration 312 comprises evaporation, the concentration 312 includes concentrating the low-salt permeate 310 in an evaporator. The evaporator used for the concentration 312 can be any type of evaporator capable of concentrating the low-salt permeate 310 to a desired solids concentration for the low-salt concentrated permeate 314. Examples of evaporators used in the concentration 312 include, but are not limited to, a rising film evaporator, a falling film evaporator, a single effect evaporator, a multiple effect evaporator, a flash evaporator, a vacuum evaporator, a centrifugal evaporator, a rotary evaporator, or a swept surface evaporator. In an example, the low-salt concentrated permeate 314 has a solids content of from about 20% to about 50% TS, for example from about 30% to about 40% TS. In some examples, the specific concentration of the low-salt concentrated permeate 314 is selected to provide for specified characteristics of the resulting final product, e.g., to provide for specified compositions or physical characteristics, or both, of the resulting syrup, gel, powder, or other form of GOS composition that results from the process.

Next, the low-salt concentrated permeate 314 is subjected to an enzyme treatment 316 to convert at least a portion of the low-salt concentrated permeate 314 to a solution 318 having a low-salt content and that includes one or more galacto-oligosaccharide compounds, referred to herein as the "low-salt GOS solution 318" for brevity. In some examples, the enzyme treatment 316 is substantially similar to the enzyme treatments 108 and 212 in the processes 100 and 200. In an example, from about 10% to about 50% of the total sugar in the low-salt GOS solution 318 after the enzyme treatment 316 is in the form of one or more galacto-oligosaccharide compounds ("GOS compounds"), such as from about 20% to about 40% of the sugars in the low-salt GOS solution 318.

In some examples, the enzyme treatment 316 is similar to the enzyme treatments 108 and 212 of the processes 100 and 200. In an example, the enzyme treatment 316 comprises exposing the low-salt concentrated permeate 314 to one or more enzymes capable of converting one or more compounds in the low-salt concentrated permeate 314 to one or more GOS compounds. In an example, the one or more enzymes of the enzyme treatment 316 comprise one or more β-galactosidase enzymes, such as one or both of the β-galactosidase from *Aspergillus oryzae* and the β-galactosidase from *Kluveromyces lactis.* In an example, the enzyme treatment 316 comprises exposing the low-salt concentrated permeate 314 to only the β-galactosidase from *Aspergillus oryzae.* In an example, the enzyme treatment 316 comprises exposing the low-salt concentrated permeate 314 to only the β-galactosidase from *Kluveromyces lactis.* In an example, the enzyme treatment 316 comprises exposing the low-salt concentrated permeate 314 to an enzyme combination comprising β-galactosidase from *Aspergillus oryzae* and β-galactosidase from *Kluveromyces lactis.*

In an example, the concentration of the one or more enzymes used in the enzyme treatment 316 is from about 0.001% to about 0.2%, by weight, such as from about 0.01% to about 0.05% by weight. In some examples, the specific relative composition of the one or more enzymes for the enzyme treatment 316, or the concentration of the one or more enzymes used in the enzyme treatment 316, or both is selected to provide for specified characteristics of the resulting final product, e.g., to provide for specified compositions or physical characteristics, or both, of the resulting syrup, gel, powder, or other form of GOS composition that results from the process. For example, if the enzyme treatment 316 is performed with a combination of the β-galactosidases from *Aspergillus oryzae* and *Kluveromyces lactis,* then the relative concentration of each enzyme in the mixture is modified to achieve different characteristics of the resulting low-salt galacto-oligosaccharide syrup 326 or low-salt galacto-oligosaccharide powder 330 (described in more detail below).

In an example, the one or more enzymes used in the enzyme treatment 316 are hydrated in a solution of potable process water prior to the enzyme treatment 316. In an example, the temperature at which the one or more enzymes is hydrated is from about 60 °F (about 15.6 °C) to about 90 °F (about 32.2 °C), such as from about 70 °F (about 21.1 °C) to about 80 °F (about 26.7 °C).

As with the enzyme treatments 108 and 212 describe above, in an example, during the enzyme treatment 316 the one or more enzymes can be allowed to react with the low-salt concentrated permeate 314 for a reaction time of from about 1 hour to about 8 hours, such as from about 2 hours to about 4 hours.

In an example, the temperature at which the enzyme treatment 316 is performed, e.g., the temperature at which the low-salt concentrated permeate 314 is exposed to the one or more enzymes, is from about 120 °F (about 48.9 °C) to about 150 °F (about 65.6 °C), such as from about 125 °F (about 51.7 °C) to about 135 °F (about 57.2 °C). In some examples, the specific temperature that the enzyme treatment 316 is performed at, or the time that the enzyme treatment 316 is performed for, or both, are selected to provide for specified characteristics of the resulting final product, e.g., to provide for specified compositions or physical characteristics, or both, of the resulting syrup, gel, powder, or other form of GOS composition that results from the process.

In some examples, after the enzyme treatment 316, the low-salt GOS solution 318 is subjected to an enzyme deactivation 320 to deactivate the one or more enzymes from the enzyme treatment 316, which provides a deactivated low-salt GOS solution 322. In an example, the enzyme deactivation 320 inhibits conversion of at least a portion of the compounds from the low-salt concentrated permeate 314 to GOS in the low-salt GOS solution 318. In an example, the enzyme deactivation 320 comprises heating the low-salt GOS solution 318 to a temperature that at least partially deactivates the one or more enzymes from the enzyme treatment 316. In some examples, the enzyme deactivation 320 comprises any heat treatment process by which the temperature of the low-salt GOS solution 318 can be raised to greater than or equal to 150 °F (about 65.6 °C) to provide a deactivated low-salt galacto-oligosaccharide solution 322, referred to herein as the "deactivated low-salt GOS solution 322" for brevity.

In some examples, after the enzyme deactivation 322, the deactivated low-salt GOS solution 322 is further concentrated, for example via evaporation 324. The evaporation 324 of the deactivated low-salt GOS solution 322 concentrates the deactivated low-salt GOS solution 322 to provide a galacto-oligosaccharide syrup 326 having a reduced salt content (hereinafter "low-salt GOS syrup 326"). In an example, the evaporation 324 concentrates the deactivated low-salt GOS syrup 326 to a solids content of from about 50% to about 75% TS, such as from about 60% to about 70% TS. In an example, the evaporation 324 is performed in any type of evaporator that is capable of concentrating the deactivated low-salt GOS solution 318 to the desired solids concentration of the low-salt GOS syrup 326. Examples of evaporators used for the evaporation 324 include, but are not limited to, a rising film evaporator, a falling film evaporator, a single effect evaporator, a multiple effect evaporator, a flash evaporator, a vacuum evaporator, a centrifugal evaporator, a rotary evaporator, or a swept surface evaporator.

In an example, the low-salt GOS syrup 326 is the final product of the process 300. In other examples, the process 300 includes further processing at least a portion of the low-salt GOS syrup 326, e.g., to provide other forms of low-salt GOS products. In an example, the process 300 includes drying at least a portion of the low-salt GOS syrup 326 to provide a solid or substantially solid galacto-oligosaccharide product having a low salt content, such as a galacto-oligosaccharide powder 330 having a low salt content, hereinafter referred to as the "low-salt GOS powder 330" for brevity.

In an example, the process 300 includes sending at least a portion of the low-salt GOS syrup 326 to a dryer 332 capable of drying the low-salt GOS syrup 326 to a moisture content level that results in the formation of the solid or substantially solid low-salt GOS product desired, e.g., the low-salt GOS powder 330. Examples of dryers used for the dryer 332 include, but are not limited to, one or more of: a pulse combustion dryer, a fluid bed dryer, a rotary dryer, a spray dryer, a roller dryer, a vacuum dryer, a box dryer, a cyclone dryer, a drum dryer, or a baghouse dryer.

In an example, the process 300 includes feeding at least a portion of the low-salt GOS syrup 326 directly into the dryer 332. In another example, the process 300 includes subjecting at least a portion of the low-salt GOS syrup 326 to a crystallization operation 334 to provide a crystallized low-salt GOS syrup 336. In an example, at least a portion of the crystallized low-salt GOS syrup 336 is fed into the dryer 332 to provide the low-salt GOS powder 330. In some examples, the crystallization 334 crystallizes one or more of, and in some examples all three of: at least a portion of the lactose in the low-salt GOS syrup 336; at least a portion of the lactose-hydrolyzed components in the low-salt GOS syrup 336; or at least a portion of the one or more GOS compounds in the low-salt GOS syrup 326.

In an example, the crystallization 334 comprises a two-stage process. In an example, the crystallization 334 includes a first crystallization stage 338 performed at a first temperature. In an example, the first temperature of the first crystallization stage 338 at least partially crystallizes at least a portion of the lactose in the low-salt GOS syrup 326 to provide a partially-crystallized low-salt GOS syrup 340. In an example, the first temperature of the first crystallization stage 338 is from about 60 °F (about 15.6 °C) to about 100 °F (about 37.8 °C).

In an example, the partially-crystallized low-salt GOS syrup 340 is subjected to a second crystallization stage 342 performed at a second temperature. In an example, the second temperature of the second crystallization stage 342 is lower than the first temperature of the first crystallization stage 338. In an example, the second temperature of the second crystallization stage 342 is from about 40 °F (about 4.4 °C) to about 80 °F (about 26.7 °C). In an example, the second crystallization stage 338 provides a fully or substantially fully crystallized low-salt GOS syrup 336. In an example, the phrase "fully or substantially fully crystallized," as used with reference to the crystallized low-salt GOS syrup 336, refers to the crystallization of one or more of the lactose, the one or more lactose-hydrolyzed components, or the one or more galacto-oligosaccharide compounds in the crystallized low-salt GOS syrup 336. In some examples, the crystallized low-salt GOS syrup 336 can include other crystallizable compounds that are uncrystallized or partially crystallized and the crystallized low-salt GOS syrup 336 will still be considered fully or substantially fully crystallized for the purposes of the process 300.

In an example, the total time of the crystallization 334 (e.g., for both the first crystallization stage 338 and the second crystallization stage 340 combined) is from about 1 hour to about 10 hours. In some examples, the first crystallization stage 338 and the second crystallization stage 340 each take about the same length of time. After the crystallization 334, at least a portion of the crystallized low-salt GOS syrup 336 is fed to the dryer 332 to provide the low-salt GOS powder 330.

The low-salt GOS syrup 326 or the crystallized low-salt GOS syrup 336 prepared by the process 300 is such that drying with just the dryer 332 (in the case of the low-salt GOS syrup 326) or with the dryer 332 after a crystallization operation, such as the crystallization 334 (in the case of the crystallized low-salt GOS syrup 336) is dried to sufficiently low moisture to provide for a powdered product, such as a flowable powder, without the use of a drying agent.

In some examples, the low-salt GOS powder 330 can have a composition of the nutritional components (e.g., galacto-oligosaccharides, glucose, galactose, lactose, protein, fat, ash, calcium, magnesium, phosphorous, sodium, chloride, and salt) that is similar or identical to that of the same nutritional components in the GOS powder 220 described above. In some examples, the low-salt GOS powder 330 is from about 20 wt.% to about 40 wt.% galacto-oligosaccharide compounds, such as from about 20 wt.% to about 30 wt.% galacto-oligosaccharide. In some examples, the low-salt GOS powder 330 is from about 10 wt.% to about 25 wt.% glucose, such as from about 15 wt.% to about 20 wt.% glucose. In some examples, the low-salt GOS powder 330 is from about 2 wt.% to about 15 wt.% galactose, such as from about 5 wt.% to about 10 wt.% galactose. In some examples, the low-salt GOS powder 330 is from about 20 wt.% to about 35 wt.% lactose, such as from about 25 wt.% to about 30 wt.% lactose. In some examples, the low-salt GOS powder 330 is from about 1 wt.% to about 7 wt.% protein, such as from about 2 wt.% to about 5 wt.% protein. In some examples, the low-salt GOS powder 330 is from about 0 wt.% to about 1.5 wt.% fat, for example from about 0 wt.% to about 1 wt.% fat. In some examples, the low-salt GOS powder 330 is from about 2 wt.% to about 14 wt.% ash, such as from about 5 wt.% to about 10 wt.% ash. In some examples, the low-salt GOS powder 330 is from about 2 wt.% to about 5 wt.% moisture, such as about 3 wt.% moisture. In some examples, the low-salt GOS powder 330 is from about 0.4 wt.% to about 0.6 wt.% calcium. In some examples, the low-salt GOS powder 330 is from about 0.1 wt.% to about 0.15 wt.% magnesium. In some examples, the low-salt GOS powder 330 is from about 0.6 wt.% to about 0.8 wt.% phosphorous. In some examples, the low-salt GOS powder 330 is from about 2 wt.% to about 5 wt.% potassium. In some examples, the low-salt GOS powder 330 is from about 0.1 wt.% to about 0.4 wt.% sodium, such as from about 0.15 wt.% to about 0.25 wt.% sodium. In some examples, the low-salt GOS powder 330 is from about 0.3 wt.% to about 0.7 wt.% potassium, such as from about 0.4 wt.% to about 0.6 wt.% potassium. In some examples, the low-salt GOS powder 330 is from about 0.1 wt.% to about 0.4 wt.% chloride, such as from about 0.15 wt.% to about 0.25 wt.% chloride. In some examples, the low-salt GOS powder 330 is from about 0.1 wt.% to about 0.6 wt.% salt, such as from about 0.2 wt.% to about 0.5 wt.% salt. In some examples, the low-salt GOS powder 330 is from about 0.4 wt.% to about 0.9 wt.% calcium, such as from about 0.5 wt.% to about 0.8% wt.% calcium. In some examples, the low-salt GOS powder 330 is from about 0.05 wt.% to about 0.4 wt.% magnesium, such as from about 0.1 wt.% to about 0.3 wt.% magnesium. In some examples, the low-salt GOS powder 330 is from about 0.3 wt.% to about 0.8 wt.% phosphorous, such as from about 0.4 wt.% to about 0.7 wt.% phosphorous.

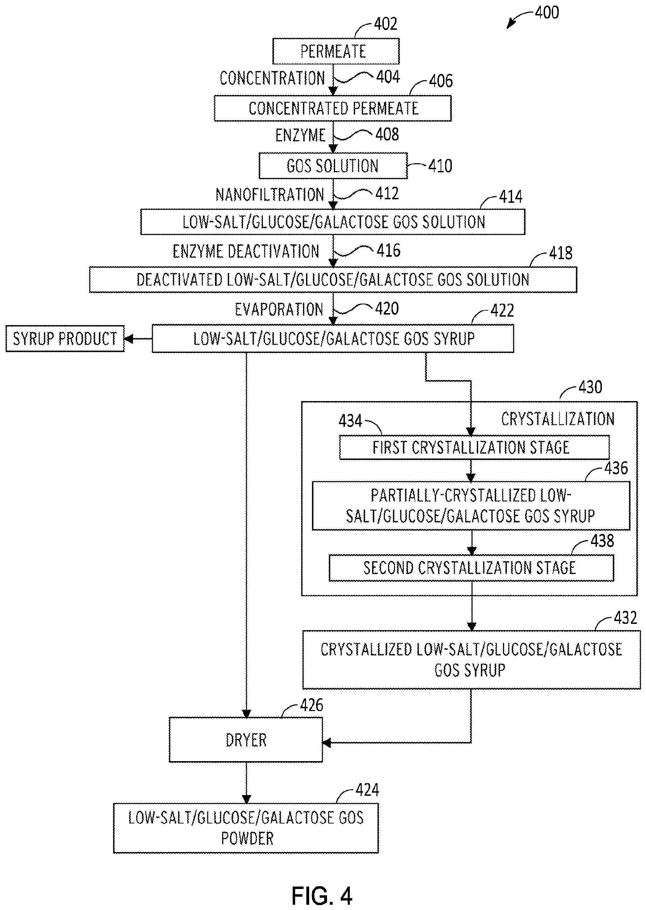

**FIG. 4** shows a flow diagram of another example process 400 for preparing a GOS product from a dairy byproduct, such as one or more permeate byproducts 402, for example at least one of a milk permeate or a whey permeate. As with the one or more permeate byproducts 102, 202, and 302 described above, the one or more permeate byproducts 402 will be referred to simply as "permeate 402" for brevity. In an example, the permeate 402 comprises a milk permeate, a whey permeate, or a mixture of a milk permeate and a whey permeate. In an example, the permeate 402 consists essentially of, or consists of, a milk permeate, a whey permeate, or a mixture of a milk permeate and a whey permeate.

The permeate 402 that is the feedstock for the process 400 can be similar to the permeate 102 described above. In an example, the permeate 402 can be identical or substantial identical to the permeate 102 described above. In an example, the permeate 402 has the same solids content as specified above with respect to the permeate 102. In an example, the permeate 402 has the same specified content of one or more components present in the permeate 402 as described above with respect to the permeate 102, e.g., the specified content of one or more of, and in some examples all of: protein, fats, lactose, ash, moisture, sodium, calcium magnesium, or potassium.

The process 400 of **FIG. 4** can be thought of as modified version of the processes 100, 200, and 300 described above, wherein the resulting GOS product is a modified or enhanced form of one or more of the GOS products 118, 122, 218, 222, 326, and 330 described with respect to processes 100, 200, and 300. In some examples, the process 400 provides a GOS product with a reduced level of salt, glucose, and galactose compared to that of the GOS products 118, 122, 218, 222, 326, and 330.

In an example, the permeate 402 is concentrated in a concentration operation 404 to provide a concentrated permeate 406 (e.g., one or both of a concentrated milk permeate or a concentrated whey permeate). In some examples, the concentration 404 of the permeate 402 is substantially similar or identical to the concentration 104, the concentration 204, or the concentration 304, described above. In an example, the concentration 304 of the permeate 302 includes one or both of reverse osmosis ("RO") or evaporation. In examples where the concentration 404 comprises RO, the concentration 404 includes concentrating the permeate 402 via the use of RO membrane filtration technology. Concentration 404 via RO can include the use of any RO membrane filtration technology capable to concentrating the permeate 402 to a desired solids content for the concentrated permeate 406. Examples of RO membrane filtration technology used for the concentration 404 include, but are not limited to, at least one of: one or more semi permeable membranes, one or more thin-film composite membranes, one or more spiral wound membranes, one or more thin sheet membranes, one or more hollow fiber membranes, one or more cellulose acetate membranes, or one or more polyamide membranes.

In examples where the concentration 404 comprises evaporation, the concentration 404 includes concentrating the permeate 402 in an evaporator. In an example, concentration 404 by evaporation provides for a desired solids content for the concentrated permeate 406. In an example, the evaporator used for the concentration 404 is any type of evaporator capable of concentrating the permeate 402 to a desired solids content for the concentrated permeate 406. Examples of evaporators used in the concentration 404 include, but are not limited to, a rising film evaporator, a falling film evaporator, a single effect evaporator, a multiple effect evaporator, a flash evaporator, a vacuum evaporator, a centrifugal evaporator, a rotary evaporator, or a swept surface evaporator. In an example, after the concentration 404, the resulting concentrated permeate 406 has a solids content of from about 10% to about 30% TS, such as from about 15% to about 25% TS, for example, about 20% TS. In some examples, the specific concentration of concentrated permeate 406 is selected to provide for specified characteristics of the resulting final product, e.g., to provide for specified compositions or physical characteristics, or both, of the resulting syrup, gel, powder, or other form of GOS composition that results from the process.

After the concentration 404, the concentrated permeate 406 is subjected to an enzyme treatment 408 to convert at least a portion of the concentrated permeate 406 to a solution 410 that includes one or more galacto-oligosaccharide compounds, referred to herein as the "GOS solution 410" for brevity. In some examples, the enzyme treatment 408 is substantially similar to the enzyme treatments 108, 212, and 316 described above with respect to processes 100, 200, and 300. In an example, from about 10% to about 50% of the total sugar in the GOS solution 410 after the enzyme treatment 408 can be in the form of one or more galacto-oligosaccharides (GOS), such as from about 20% to about 40% of the sugars in the GOS solution 410.

In some examples, the enzyme treatment 408 comprises exposing the concentrated permeate 406 to one or more enzymes capable of converting one or more compounds in the concentrated permeate 406 to one or more GOS compounds. In an example, the one or more enzymes of the enzyme treatment 408 comprise one or more β-galactosidase enzymes, such as one or both of the β-galactosidase from *Aspergillus oryzae* and the β -galactosidase from *Kluveromyces lactis.* In an example, the enzyme treatment 408 comprises exposing the concentrated permeate 406 to only the β-galactosidase from *Aspergillus oryzae.* In an example, the enzyme treatment 408 comprises exposing the concentrated permeate 406 to only the β-galactosidase from *Kluveromyces lactis.* In an example, the enzyme treatment 408 comprises exposing the concentrated permeate 406 to an enzyme combination comprising β-galactosidase from *Aspergillus oryzae* and β-galactosidase from *Kluveromyces lactis.*

In an example, the concentration of the one or more enzymes used in the enzyme treatment 408 can be from about 0.001 to about 0.2%, by weight, such as from about 0.01% to about 0.05% by weight. In some examples, the specific relative composition of the one or more enzymes for the enzyme treatment 408, or the concentration of the one or more enzymes used in the enzyme treatment 408, or both is selected to provide for specified characteristics of the resulting final product, e.g., to provide for specified compositions or physical characteristics, or both, of the resulting syrup, gel, powder, or other form of GOS composition that results from the process. For example, if the enzyme treatment 408 is performed with a combination of the β-galactosidases from *Aspergillus oryzae* and *Kluveromyces lactis,* then the concentration of each enzyme in the mixture is modified to achieve different characteristics of the resulting low-salt, low-glucose, low-galactose galacto-oligosaccharide syrup 422 or low-salt, low-glucose, low galactose galacto-oligosaccharide powder 424 (described in more detail below).

In an example, the one or more enzymes used in the enzyme treatment 408 are hydrated in a solution of potable process water prior to the enzyme treatment 408. In an example, the temperature at which the one or more enzymes is hydrated is from about 60 °F (about 15.6 °C) to about 90 °F (about 32.2 °C), such as from about 70 °F (about 21.1 °C) to about 80 °F (about 26.7 °C).

As with the enzyme treatments 108, 212, and 316, in an example, during the enzyme treatment 408 the one or more enzymes can be allowed to react with the concentrated permeate 406 for a reaction time of from about 1 hour to about 8 hours, such as from about 2 hours to about 4 hours. In an example, the temperature at which the enzyme treatment 408 is performed, e.g., the temperature at which the concentrated permeate 406 is exposed to the one or more enzymes, can be from about 120 °F (about 48.9 °C) to about 150 °F (about 65.6 °C), such as from about 125 °F (about 51.7 °C) to about 135 °F (about 57.2 °C). In some examples, the specific temperature that the enzyme treatment 408 is performed at, or the time that the enzyme treatment 408 is performed for, or both, are selected to provide for specified characteristics of the resulting final product, e.g., to provide for specified compositions or physical characteristics, or both, of the resulting syrup, gel, powder, or other form of GOS composition that results from the process.

In an example, after the enzyme treatment 408, the GOS solution 410 is subjected to nanofiltration 412 to remove monovalent salt compounds from the GOS solution 410. Examples of monovalent salts that are removed from the GOS solution 410 by the nanofiltration 412 (described in more detail below) include, but are not limited to, one or more of sodium chloride (NaCl) or potassium chloride (KCl). In an example, the nanofiltration 412 of the GOS solution 410 removes sugars other than the GOS compounds, such as a glucose and galactose. In an example, after the nanofiltration 412, a galacto-oligosaccharide solution 414 having a reduced content of salt, glucose, and galactose can result, hereinafter referred to as the "low-salt, low-glucose, low-galactose GOS solution 414," or simply the "low-salt/glucose/galactose GOS solution 414" for brevity.

In an example, the nanofiltration 412 (hereinafter "NF 412") includes passing the GOS solution 410 through a nanofiltration membrane. In some examples, the nanofiltration membrane removes salts, glucose, and galactose from the GOS solution 410 in order to provide the low-salt/glucose/galactose GOS solution 414. In this way, the NF 412 of the process 400 is similar to the NF 308 of the process 400, except that the nanofiltration membrane removes not only at least a portion of the salts, but also at least a portion of the glucose and galactose that are produced during the enzyme treatment 408.

In an example, the NF 412 reduces salt in the GOS solution 410, on a dry weight basis, by at least about 50%, such as at least about 60%, for example at least about 75%, such as at least about 80%, and in some examples by about 89% or 90% or more. In an example, the NF 412 results in similar or comparable reduction in sodium. In an example, shown in Table 2 (described in more detail below), the NF 412 reduces the dry-basis salt content by about 89%, from about 2.8 g/100 g dry product to about 0.3 g/100g in the low-salt/glucose/galactose GOS solution 414. As shown in Table 3 (described in more detail below), in an example, the NF 412 results in a reduction of the sodium, on a dry weight basis, of about 80%, from about 0.7 g/100 g dry product to about 0.14 g/100 g in the low-salt/glucose/galactose GOS solution 414. In an example, the NF 412 results in reduction of other components from the GOS solution 410, such as other minerals (e.g., potassium, chloride, and to a lesser extent, phosphorus) and non-protein nitrogen (NPN).

In some examples, the NF 412 reduces sugars in the GOS solution 410 that are produced during the enzyme treatment 408, such as glucose and galactose. In an example, the NF 412 reduces glucose in the GOS solution 410, on a dry weight basis, by at least about 30%, such as at least about 50%, for example at least about 60%, such as about 62.5% or more. In an example, the NF 412 reduces galactose in the GOS solution 410, on a dry weight basis, by at least about 30%, for example at least about 50%, such as about 55% or more. In an example, shown in Table 2 (described in more detail below), the NF 412 reduces the dry-basis glucose content by about 62.5%, from about 16 g/100 g dry product to about 6 g/100g in the low-salt/glucose/galactose GOS solution 414. As further shown in the examples of Table 2, the NF 412 reduces the dry-basis galactose content by about 55%, from about 4.5 g/100 g dry product to about 2 g/100g in the low-salt/glucose/galactose GOS solution 414.

As described above, in some examples, the NF 412 provides for the selective rejection of salts and sugars from the GOS solution 410. The NF 412 is performed using any type of nanofiltration ("NF") process that is capable of achieving desired levels of salt, glucose, and galactose to provide the low-salt/glucose/galactose GOS solution 414. In some examples, the NF 412 utilizes NF membrane filtration technology. Examples of NF membrane filtration technology used for the NF 412 include, but are not limited to, semi-permeable membranes, thin-film composite membranes, spiral-wound membranes, thin-sheet membranes, hollow-fiber membranes, cellulose acetate membranes, or polyamide membranes.

In some examples, after the NF 412 that provides the low-salt/glucose/galactose GOS solution 414, the low-salt/glucose/galactose GOS solution 414 can be subjected to an enzyme deactivation 416 to deactivate the one or more enzymes from the enzyme treatment 408. In an example, the enzyme deactivation 416 inhibits conversion of a least a portion of the compounds from the concentrated permeate 406 to GOS compounds. The enzyme deactivation 416 provides a GOS solution 418 with deactivated enzymes and reduced levels of salt, glucose, and galactose, referred to herein as the "deactivated low-salt, low-glucose, low-galactose GOS solution 418," or simply the "deactivated low-salt/glucose/galactose GOS solution 418" for brevity. In some examples, the enzyme deactivation 416 includes any heat treatment process by which the temperature of the low-salt/glucose/galactose GOS solution 414 can be raised to a temperature greater than or equal to 150 °F (about 65.6 °C).

In some examples, after the enzyme deactivation 426, the deactivated low-salt/glucose/galactose GOS solution 418 is concentrated, for example via evaporation 420. In some examples, the evaporation 420 is performed under conditions that are sufficient to deactivate the one or more enzymes used in the enzyme treatment 408. In an example, the evaporation 420 concentrates the deactivated low-salt/glucose/galactose GOS solution 418 to provide a galacto-oligosaccharide syrup 422 having reduced levels of salt, glucose, and galactose, referred to herein as the "low-salt/glucose/galactose GOS syrup 422" for brevity. In an example, the evaporation 420 concentrates the low-salt/glucose/galactose GOS syrup 422 to a solids content of from about 50% to about 75% TS, such as from about 60% to about 70% TS. In an example, the evaporation 420 is performed in any type of evaporator that is capable of concentrating the deactivated low-salt/glucose/galactose GOS solution 418 to the desired solids concentration of the low-salt/glucose/galactose GOS syrup 422, including, but not limited to, a rising film evaporator, a falling film evaporator, a single effect evaporator, a multiple effect evaporator, a flash evaporator, a vacuum evaporator, a centrifugal evaporator, a rotary evaporator, or a swept surface evaporator.

In an example, the low-salt/glucose/galactose GOS syrup 422 is the final product of the process 400. In other examples, the process 400 includes further processing of the low-salt/glucose/galactose GOS syrup 422, e.g., to provide other forms of low-salt, low-glucose, and low-galactose products. In an example, the process 400 includes drying the low-salt/glucose/galactose GOS syrup 422 to provide a solid or substantially solid galacto-oligosaccharide powder 426, having a reduced salt content, a reduced glucose content, and a reduced galactose content, referred to herein as the "low-salt, low-glucose, and low-galactose GOS powder 426," or simply the "low-salt/glucose/galactose GOS powder 426" for brevity.

In an example, the process 400 includes sending at least a portion of the low-salt/glucose/galactose GOS syrup 422 to a dryer 428 capable of drying the low-salt/glucose/galactose GOS syrup 422 to a moisture content level that results in the formation of the solid or substantially solid low-salt, low-glucose, and low-galactose product desired, e.g., the low-salt/glucose/galactose GOS powder 426. Examples of dryers used for the dryer 428 include, but are not limited to, one or more of: a pulse combustion dryer, a fluid bed dryer, a rotary dryer, a spray dryer, a roller dryer, a vacuum dryer, a box dryer, a cyclone dryer, a drum dryer, or a baghouse dryer.

In an example, the process 400 includes feeding at least a portion of the low-salt/glucose/galactose GOS syrup 422 directly into the dryer 428. In another example, the process 400 includes subjecting at least a portion of the low-salt/glucose/galactose GOS syrup 422 to a crystallization operation 430 to provide an at least partially crystallized low-salt/glucose/galactose GOS syrup 432. In an example, at least a portion of the crystallized low-salt/glucose/galactose GOS syrup 432 is fed into the dryer428 to provide the low-salt/glucose/galactose powder 426. In some examples, the crystallization 430 crystallizes one or more of, and in some examples all three of: at least a portion of the lactose in the low-salt/glucose/galactose GOS syrup 422; at least a portion of the lactose-hydrolyzed components in the low-salt/glucose/galactose GOS syrup 422; or at least a portion of the one or more GOS compounds in the low-salt/glucose/galactose GOS syrup 422.

In an example, the crystallization 430 comprises a two-stage process. In an example, the crystallization 430 includes a first crystallization stage 434 performed at a first temperature. In an example, the first temperature of the first crystallization stage 434 at least partially crystallizes at least a portion of the lactose in the low-salt/glucose/galactose GOS syrup 422 to provide a partially-crystallized low-salt/glucose/galactose GOS syrup 436. In an example, the first temperature of the first crystallization stage 434 is from about 60 °F (about 15.6 °C) to about 100 °F (about 37.8 °C).

In an example, the partially-crystallized low-salt/glucose/galactose GOS syrup 436 is subjected to a second crystallization stage 438 performed at a second temperature. In an example, the second temperature of the second crystallization stage 438 is lower than the first temperature of the first crystallization stage 434. In an example, the second temperature of the second crystallization stage 438 is from about 40 °F (about 4.4 °C) to about 80 °F (about 26.7 °C). In an example, the second crystallization stage 438 provides a fully or substantially fully crystallized low-salt/glucose/galactose GOS syrup 432. In an example, the phrase "fully or substantially fully crystallized," as used with reference to the crystallized low-salt/glucose/galactose GOS syrup 432, refers to the crystallization of one or more of the lactose, the one or more lactose-hydrolyzed components, or the one or more galacto-oligosaccharide compounds in the crystallized low-salt/glucose/galactose GOS syrup 432. In some examples, the crystallized low-salt/glucose/galactose GOS syrup 432 can include other crystallizable compounds that are uncrystallized or partially crystallized and the crystallized low-salt/glucose/galactose GOS syrup 432 will still be considered fully or substantially fully crystallized for the purposes of the process 400.

In an example, the total time of the crystallization 430 (e.g., for both the first crystallization stage 434 and the second crystallization stage 438 combined) is from about 1 hour to about 10 hours. In some examples, the first crystallization stage 434 and the second crystallization stage 438 each take about the same length of time. After the crystallization 430, at least a portion of the crystallized low-salt/glucose/galactose GOS syrup 432 is fed to the dryer 428 to provide the low-salt/glucose/galactose GOS powder 426.

The low-salt/glucose/galactose GOS syrup 422 or the crystallized low-salt/glucose/galactose GOS syrup 432 prepared by the process 400 is such that drying with just the dryer 428 (in the case of the low-salt/glucose/galactose GOS syrup 422) or with the dryer 428 after a crystallization operation, such as the crystallization 430 (in the case of the crystallized low-salt/glucose/galactose GOS syrup 432) is dried to sufficiently low moisture to provide for a powdered product (e.g., the low-salt/glucose/galactose GOS powder 426) such as a flowable powder, without the use of a drying agent.

In some examples, the low-salt/glucose/galactose GOS powder 426 can have a composition of the nutritional components (e.g., galacto-oligosaccharides, glucose, galactose, lactose, protein, fat, ash, calcium, magnesium, phosphorous, sodium, chloride, and salt) that is similar or identical to that of the same nutritional components in the GOS powder 220 described above. In some examples, the low-salt/glucose/galactose GOS powder 426 is from about 20 wt.% to about 40 wt.% galacto-oligosaccharide compounds, such as from about 20 wt.% to about 30 wt.% galacto-oligosaccharide. In some examples, the low-salt/glucose/galactose GOS powder 426 is from about 2 wt.% to about 10 wt.% glucose, such as from about 4 wt.% to about 8 wt.% glucose. In some examples, the low-salt/glucose/galactose GOS powder 426 is from about 1 wt.% to about 5 wt.% galactose, such as from about 2 wt.% to about 4 wt.% galactose. In some examples, the low-salt/glucose/galactose GOS powder 426 is from about 30 wt.% to about 50 wt.% lactose, such as from about 35 wt.% to about 45 wt.% lactose. In some examples, the low-salt/glucose/galactose GOS powder 426 is from about 1 wt.% to about 4 wt.% protein, such as from about 2 wt.% to about 3 wt.% protein. In some examples, the low-salt/glucose/galactose GOS powder 426 is from about 0 wt.% to about 0.5 wt.% fat, for example from about 0 wt.% to about 0.3 wt.% fat. In some examples, the low-salt/glucose/galactose GOS powder 426 is from about 2 wt.% to about 8 wt.% ash, such as from about 4 wt.% to about 6 wt.% ash. In some examples, the low-salt/glucose/galactose GOS powder 426 is from about 2 wt.% to about 5 wt.% moisture, such as about 3 wt.% moisture. In some examples, the low-salt/glucose/galactose GOS powder 426 is from about 0.3 wt.% to about 0.9 wt.% potassium, such as from about 0.4 wt.% to about 0.8 wt.% potassium. In some examples, the low-salt/glucose/galactose GOS powder 426 is from about 0.05 wt.% to about 0.4 wt.% sodium, such as from about 0.1 wt.% to about 0.3 wt.% sodium. In some examples, the low-salt/glucose/galactose GOS powder 426 is from about 0.05 wt.% to about 0.4 chloride, such as from about 0.1 wt.% to about 0.3 wt.% chloride. In some examples, the low-salt/glucose/galactose GOS powder 426 is from about 0.1 wt.% to about 0.6 wt.% salt, such as from about 0.2 wt.% to about 0.5 wt.% salt.

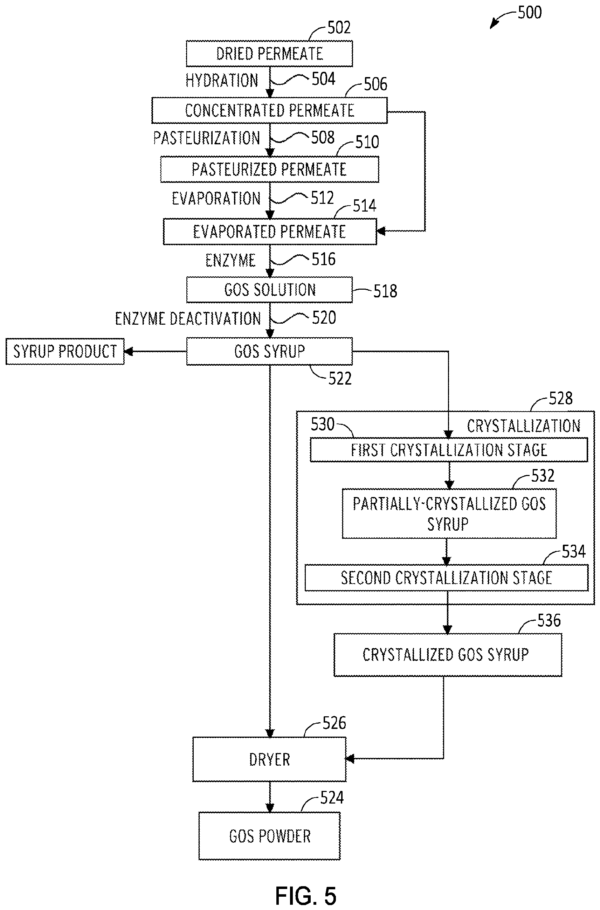

**FIG. 5** shows a flow diagram of another example process 500 for preparing a GOS product from a dairy byproduct, such as one or more permeate byproducts 502, for example at least one of a milk permeate or a whey permeate. As with the one or more permeate byproducts 102, 202, 302, and 402 described above in the process 100, 200, 300, and 400 of **FIG. 1****,** **2****,** **3****, and** **4****,** the one or more permeate byproducts 502 will be referred to simply as "permeate 502" for brevity. In an example, the permeate 502 comprises a milk permeate, a whey permeate, or a mixture of a milk permeate and a whey permeate. In an example, the permeate 502 consists essentially of, or consists of, a milk permeate, a whey permeate, or a mixture of a milk permeate and a whey permeate.

The permeate 502 that is the feedstock for the process 500 can be similar to the permeate 102 described above for the process 100. In an example, the compositional profile of the permeate 502 can be identical or substantial identical to the permeate 102 described above, with the exception that the permeate 502 is in the form of a dried powder and in an example has a solids contents of 94% or more. In an example, the permeate 502 has the same specified content of one or more components present in the permeate 102 as described above with respect to the permeate 102, e.g., the specified content of one or more of, and in some examples all of: protein, fats, lactose, ash, sodium, calcium magnesium, or potassium.

The process 500 of **FIG. 5** can be thought of as an alternative to that of process 200, with the process 500 producing a GOS product in similar forms of the GOS syrup 218, or the GOS powder 220 that result from the process 200.

In an example, the permeate 502 is changed from a dried powder form to a liquid form by the addition of potable water to the permeate 502. In an example, the permeate 502 is subjected to hydration 504. In an example, hydration 504 thereby provides a concentrated permeate 506, having a solids content of from about 10% to about 70% TS, such as from 20% to about 60% TS. In an example, hydration 504 can be accomplished by any type of process equipment capable of hydrating the permeate 502 to a desired solids concentration for the concentrated permeate 506. Examples of process equipment used in the hydration 504 include, but are not limited to, a tank and/or silo with any form of agitation, a reconstituting system, a blending system, a blending pump, a mixing pump, a shear mixing pump, a mixing pump and/or system under vacuum, or any system capable of blending water and dried powders.

In some examples, after hydration 504, the concentrated permeate 506 is further processed via a pasteurization operation 508 to provide a pasteurized permeate 510. In an example, pasteurization 508 provides a pasteurized permeate 510 that compromises a reduced micro bacterial composition, as compared to the micro bacterial composition of the permeate 502 and the concentrated permeate 506. In some examples, the pasteurized permeate 510 will have a significantly lower total plate count, standard plate count, coli form count, content of salmonella, content of listeria, or content of E. coli. Examples of process equipment used in the pasteurization 508 include, but are not limited to, a batch or vat pasteurizer, whereby the concentrated permeate 506 is held in a vessel and heated to a specific temperature for a specified duration, a high-temperature, short-time pasteurizer, also known as an HTST, an ultrahigh temperature pasteurizer, also known as a UHT, or any process whereby the concentrated permeate 506 is heated to an increased temperature in an effort to reduce the micro bacteria or pathogen content of the permeate 502 and the concentrated permeate 506, and also includes any such process that can or cannot be defined as a "legal" pasteurization step, as defined by state or federal agencies that govern the pasteurization requirements for dairy products.

In some examples, after the pasteurization 508, the pasteurized permeate 510 is further concentrated, such as via an evaporation operation 512 to provide an evaporated permeate 514. In an example, the evaporated permeate 514 has a solids content of from about 50% to about 80% TS, such as from about 60% to about 70% TS. In some examples, the specific concentration of the evaporated permeate 514 is selected to provide for specified characteristics of the resulting final product, e.g., to provide for specified compositions or physical characteristics, or both, of the resulting syrup, gel, powder, or other form of GOS composition that results from the process.

In an example, the evaporation 512 is performed in any type of evaporator that is capable of concentrating the pasteurized permeate 510 to the desired solids concentration for the evaporated permeate 514. Examples of evaporators that can be used for the evaporation 512 include, but are not limited to, a rising film evaporator, a falling film evaporator, a single effect evaporator, a multiple effect evaporator, a flash evaporator, a vacuum evaporator, a centrifugal evaporator, a rotary evaporator, or a swept surface evaporator.

Next, the evaporated permeate 514 is subjected to an enzyme treatment 516 to convert at least a portion of the evaporated permeate 514 to a solution 518 including one or more galacto-oligosaccharide compounds, referred to herein as the "GOS solution 518" for the sake of brevity. In some examples, the enzyme treatment 516 is substantially similar to the enzyme treatment 108, except that it is being performed on the evaporated permeate 514, which has a higher solids content than the concentrated permeate 106 in the process 100. In an example, from about 10% to about 50% of the total sugar in the GOS solution 518 after the enzyme treatment 516 is in the form of one or more galacto-oligosaccharides (GOS), such as from about 20% to about 30% of the sugars in the GOS solution 518.

In an example, the enzyme treatment 516 comprises exposing the evaporated permeate 514 to one or more enzymes capable of converting one or more compounds in the evaporated permeate 514 to one or more GOS compounds, similar to the enzyme treatment 108. In an example, the one or more enzymes comprise one or more β-galactosidase enzymes, such as one or both of the β-galactosidase from *Aspergillus oryzae* and the β-galactosidase from *Kluveromyces lactis.* In an example, the enzyme treatment 516 comprises exposing the evaporated permeate 514 to only the β-gaiactosidase from *Aspergillus oryzae.* In an example, the enzyme treatment 516 comprises exposing the evaporated permeate 514 to only the β-galactosidase from *Kluveromyces lactis.* In an example, the enzyme treatment 516 comprises exposing the evaporated permeate 514 to an enzyme combination comprising β-galactosidase from *Aspergillus oryzae* and β-galactosidase from *Kluveromyces lactis.*

In an example, the concentration of the one or more enzymes used in the enzyme treatment 516 is from about 0.001% to about 0.2%, by weight, such as from about 0.01% to about 0.05% by weight. In some examples, the specific relative composition of the one or more enzymes for the enzyme treatment 516, or the concentration of the one or more enzymes used in the enzyme treatment 516, or both is selected to provide for specified characteristics of the resulting final product, e.g., to provide for specified compositions or physical characteristics, or both, of the resulting syrup, gel, powder, or other form of GOS composition that results from the process. For example, if the enzyme treatment 516 is performed with a combination of the β-galactosidases from *Aspergillus oryzae* and *Kluveromyces lactis*, then the concentration of each enzyme in the mixture is modified to achieve different characteristics of the resulting galacto-oligosaccharide syrup 522 or galacto-oligosaccharide powder 524 (described in more detail below).

In an example, the one or more enzymes is hydrated in a solution of potable process water prior to the enzyme treatment 516. In an example, the temperature at which the one or more enzymes is hydrated is from about 60 °F (about 15.6 °C) to about 90 °F (about 32.2 °C), such as from about 70 °F (about 21.1 °C) to about 80 °F (about 26.7 °C).

As with the enzyme treatment 108 that provides the GOS solution 110, in an example, during the enzyme treatment 516, the one or more enzymes are allowed to react with the evaporated permeate 514 for a reaction time of from about 1 hour to about 8 hours, such as from about 2 hours to about 4 hours. In an example, the temperature at which the enzyme treatment 516 is performed, e.g., the temperature at which the evaporated permeate 514 is exposed to the one or more enzymes, is from about 120 °F (about 48.9 °C) to about 150 °F (about 65.6 °C), such as from about 125 °F (about 51.7 °C) to about 135 °F (about 57.2 °C). In some examples, the specific temperature that the enzyme treatment 516 is performed at, or the time that the enzyme treatment 516 is performed for, or both, are selected to provide for specified characteristics of the resulting final product, e.g., to provide for specified compositions or physical characteristics, or both, of the resulting syrup, gel, powder, or other form of GOS composition that results from the process.

In some examples, after the enzyme treatment 516 that provides the GOS solution 518, the GOS solution 518 is subjected to an enzyme deactivation 520 to deactivate the one or more enzymes from the enzyme treatment 516. In an example, the enzyme deactivation 520 inhibits conversion of at least a portion of the compounds from the evaporated permeate 514 to GOS in the GOS solution 518. In an example, the enzyme deactivation 520 comprises heating the GOS solution 518 to a temperature that at least partially deactivates the one or more enzymes from the enzyme treatment 516. In an example, the enzyme deactivation 520, in addition to deactivating the one or more enzymes from the enzyme treatment 516, includes concentrating the GOS solution 518 by evaporation to provide a galacto-oligosaccharide syrup 522 (hereinafter referred to as a "GOS syrup 522"). In an example, the evaporation is performed under conditions that are sufficient to deactivate the one or more enzymes used in the enzyme treatment 516. In an example, the enzyme deactivation 520 comprises any heat treatment process by which the temperature of the GOS solution 518 is raised to greater than or equal to 150 °F (about 65.6 °C) to provide the GOS syrup 522.

In an example, the GOS syrup 522 is the final product of the process 500. In other examples, the process 500 includes further processing of the GOS syrup 522, e.g., to provide other forms of GOS products. In an example, the process 500 includes drying the GOS syrup 522 to provide a solid or substantially solid galacto-oligosaccharide product, such as a galacto-oligosaccharide powder 524 (hereinafter "GOS powder 524"). In an example, the process 500 includes sending at least a portion of the GOS syrup 522 to a dryer 526 capable of drying the GOS syrup 522 to a moisture content level that results in the formation of the solid or substantially solid GOS product desired, e.g., the GOS powder 524. Examples of dryers 526 used to for drying the GOS syrup 522 to form the GOS powder 524 include, but are not limited to, one or more of a pulse combustion dryer, a fluid bed dryer, a rotary dryer, a spray dryer, a roller dryer, a vacuum dryer, a box dryer, a cyclone dryer, a drum dryer, or a baghouse dryer.

In an example, the process 500 includes feeding at least a portion of the GOS syrup 522 directly into the dryer 526. In another example, the process 500 includes subjecting at least a portion of the GOS syrup 522 to a crystallization operation 528 to provide a crystallized GOS syrup 536. In an example, at least a portion of the crystallized GOS syrup 536 is fed into the dryer 526 to produce the GOS powder 524. In some examples, the crystallization 528 crystallizes one or more of, and in some examples all three of: at least a portion of the lactose in the GOS syrup 522; at least a portion of lactose-hydrolyzed components in the GOS syrup 522; or at least a portion of one or more GOS compounds in the GOS syrup 522.

In an example, the crystallization 528 comprises a two-stage process. In an example, the crystallization 528 includes a first crystallization stage 530 performed at a first temperature. In an example, the first temperature of the first crystallization stage 530 at least partially crystallizes at least a portion of the lactose in the GOS syrup 522 to provide a partially-crystallized GOS syrup 532. In an example, the first temperature of the first crystallization stage 530 is from about 60 °F (about 15.6 °C) to about 100 °F (about 37.8 °C).

In an example, the partially crystallized GOS syrup 532 is subjected to a second crystallization stage 534 performed at a second temperature. In an example, the second temperature of the second crystallization stage 534 is lower than the first temperature of the first crystallization stage 530. In an example, the second temperature of the second crystallization stage 534 is from about 40 °F (about 4.4 °C) to about 80 °F (about 26.7 °C). In an example, the second crystallization stage 534 provides a fully or substantially fully crystallized GOS syrup 536. In an example, the phrase "fully or substantially fully crystallized," as used with reference to the crystallized GOS syrup 536, refers to the crystallization of one or more of the lactose, the one or more lactose-hydrolyzed components, or the one or more galacto-oligosaccharide compounds in the crystallized GOS syrup 536. In some examples, the crystallized GOS syrup 536 can include other crystallizable compounds that are uncrystallized or partially crystallized and the crystallized GOS syrup 536 will still be considered fully or substantially fully crystallized for the purposes of the process 500.

In an example, the total time of the crystallization 528 (e.g., for both the first crystallization stage 530 and the second crystallization stage 534 combined) is from about 1 hour to about 10 hours. In some examples, the first crystallization stage 530 and the second crystallization stage 534 each take about the same length of time. After the crystallization 528, at least a portion of the crystallized GOS syrup 536 is fed to the dryer 526 to provide the GOS powder 524.

In an example, the drying of the GOS syrup 522 or the crystallized GOS syrup 536 is performed without the use of a drying agent. As used herein, the term "drying agent" refers to a compound or mixture of compounds that is contacted with a composition to be dried, such as the GOS syrup 522 or the crystallized GOS syrup 536, in order to aid in the removal of moisture from the composition for the purposes of drying that composition. In some previous milk and whey byproduct processing, drying of the final products to form a powder, and in particular a flowable powder, has been difficult or even impossible without the use of a drying agent. Examples of drying agents that have been used to form powdered products from dairy operations, including from milk and whey byproducts, have included milk proteins, caseinate, whey proteins, nonfat dry milk, skim milk, lactose, tricalcium phosphate, dicalcium phosphate, kaolin, diatomaceous earth, silica, calcium silicate hydrate, maltodextrins, and starches, or mixtures thereof. The GOS syrup 522 or the crystallized GOS syrup 536 prepared by the process 500 is such that drying with just the dryer 526 (in the case of the GOS syrup 522) or with the dryer 526 after a crystallization operation, such as the crystallization 528 (in the case of the crystallized GOS syrup 536) can be dried to sufficiently low moisture to provide for a powdered product, such as a flowable powder, without the use of a drying agent.

In some examples, the GOS powder 524 is from about 20 wt.% to about 40 wt.% galacto-oligosaccharide compounds, such as from about 20 wt.% to about 30 wt.% galacto-oligosaccharide. In some examples, the GOS powder 524 is from about 10 wt.% to about 25 wt.% glucose, such as from about 15 wt.% to about 20 wt.% glucose. In some examples, the GOS powder 524 is from about 2 wt.% to about 15 wt.% galactose, such as from about 5 wt.% to about 10 wt.% galactose. In some examples, the GOS powder 524 is from about 20 wt.% to about 35 wt.% lactose, such as from about 25 wt.% to about 30 wt.% lactose. In some examples, the GOS powder 524 is from about 1 wt.% to about 7 wt.% protein, such as from about 2 wt.% to about 5 wt.% protein. In some examples, the GOS powder 524 is from about 0 wt.% to about 1.5 wt.% fat, for example from about 0 wt.% to about 1 wt.% fat. In some examples, the GOS powder 524 is from about 2 wt.% to about 14 wt.% ash, such as from about 5 wt.% to about 10 wt.% ash. In some examples, the GOS powder 524 is from about 2 wt.% to about 5 wt.% moisture, such as about 3 wt.% moisture. In some examples, the GOS powder 524 is from about 0.4 wt.% to about 0.6 wt.% calcium. In some examples, the GOS powder 524 is from about 0.1 wt.% to about 0.15 wt.% magnesium. In some examples, the GOS powder 524 is from about 0.6 wt.% to about 0.8 wt.% phosphorous. In some examples, the GOS powder 524 is from about 2 wt.% to about 5 wt.% potassium. In some examples, the GOS powder 524 is from about 0.2 wt.% to about 1 wt.% sodium, such as from about 0.4 wt.% to about 0.9 wt.% sodium. In some examples, the GOS powder 524 is from about 0.5 wt.% to about 1.8 chloride, such as from about 0.6 wt.% to about 1 wt.% chloride. In some examples, the GOS powder 524 is from about 1 wt.% to about 4 wt.% salt, such as from about 2 wt.% to about 3 wt.% salt.

### EXAMPLES

The present disclosure can be better understood by reference to the Example which is offered by way of illustration. The present disclosure is not limited to the Example given herein.

### EXAMPLE 1

A permeate feedstock having a solids content of 6.7% TS was subjected to reverse osmosis to provide a permeate having a solids content of 20% TS. The 20% TS permeate was then further concentrated by evaporation to provide a permeate having a solids content of 40% TS.

The 40% TS permeate was exposed to an enzyme mixture of the β-galactosidase from *Aspergillus oryzae* and the β-gaiactosidase from *Kluveromyces lactis.* The enzymes were hydrated with potable process water at a temperature of about 70 °F (about 21.1 °C) to about 80 °F (about 26.7 °C). The enzyme concentration after mixing the enzyme mixture with the 40% TS permeate was about 0.01% to about 0.05% by weight. The 40% TS permeate was allowed to react with the enzyme mixture for about 2 hours to about 4 hours at a temperature of from about 125 °F (51.7 °C) to about 135 °F (57.2 °C).

The enzyme treatment resulted in a galacto-oligosaccharide solution ("GOS solution") having a solids content of about 40% TS. Two batches of the GOS solution were concentrated by evaporation to provide two batches of galacto-oligosaccharide syrup ("GOS syrup"), with the first batch of GOS syrup having a solids content of about 60% TS and the second batch of GOS syrup having a solids content of about 70% TS. A third batch of the GOS solution was concentrated by evaporation to a galacto-oligosaccharide gel ("GOS gel") having a solids content of about 80% TS. The evaporation to form the GOS syrup batches and the GOS gel batch also deactivated the enzymes from the enzyme mixture

### EXAMPLE 2

The same permeate feedstock as Example 1 was subjected to reverse osmosis to provide a permeate having a solids content of 20% TS. The 20% TS permeate was then further concentrated by evaporation to provide a concentrated permeate having a solids content of 60% TS.

The 60% TS permeate was exposed to an enzyme mixture of the β-galactosidase from *Aspergillus oryzae* and the β-galactosidase from *Kluveromyces lactis.* The enzymes were hydrated with potable process water at a temperature of about 70 °F (about 21.1 °C) to about 80 °F (about 26.7 °C). The enzyme concentration after mixing the enzyme mixture with the 60% TS permeate was about 0.01% to about 0.05% by weight. The 60% TS permeate was allowed to react with the enzyme mixture for about 2 hours to about 4 hours at a temperature of from about 125 °F (51.7 °C) to about 135 °F (57.2 °C). The enzyme treatment resulted in a galacto-oligosaccharide syrup ("GOS syrup") having a solids content of about 60% TS.

The enzymes in the 60% TS GOS syrup were deactivated by heating the GOS syrup to a temperature above 150 °F (about 65.6 °C). The GOS syrup was then subjected to a crystallization cycle including a first crystallization stage of cooling the GOS syrup to a temperature of 60 °F (15.6 °C) to about 80 °F (about 26.7 °C) to crystallize lactose present in the GOS syrup. The GOS syrup was then subjected to a second crystallization stage by cooling the GOS syrup to a temperature from 40 °F (about 4.4 °C) to about 60 °F (about 15.6 °C).

The crystallized GOS syrup was then dried in a spray dryer at a feed concentration of about 40% TS. The inlet temperature of the spray dryerwas held at about 240 °F (115.6 °C). The outlet temperature of the spray dryer was maintained at about 180 °F (82.2 °C). The spray drying resulted in a galacto-oligosaccharide powder ("GOS powder").

### EXAMPLE 3

The same permeate feedstock as Examples 1 and 2 was subjected to reverse osmosis to provide a permeate having a solids content of 20% TS. The 20% TS permeate was then further concentrated by evaporation to provide a concentrated permeate having a solids content of 70% TS.

The 70% TS permeate was exposed to an enzyme mixture of the β-galactosidase from *Aspergillus oryzae* and the β-gaiactosidase from *Kluveromyces lactis.* The enzymes were hydrated with potable process water at a temperature of about 70 °F (about 21.1 °C) to about 80 °F (about 26.7 °C). The enzyme concentration after mixing the enzyme mixture with the 70% TS permeate was about 0.01% to about 0.05% by weight. The 70% TS permeate was allowed to react with the enzyme mixture for about 2 hours to about 4 hours at a temperature of from about 125 °F (51.7 °C) to about 135 °F (57.2 °C).

The enzyme treatment resulted in a galacto-oligosaccharide syrup ("GOS syrup") having a solids content of about 70% TS. The enzymes in the GOS syrup were deactivated by heating the GOS syrup to a temperature above 150 °F (about 65.6 °C).

The GOS syrup was then dried in a spray dryer at a feed concentration of about 40% TS. The inlet temperature of the spray dryer was held at about 240 °F (115.6 °C).The outlet temperature of the spray dryer was maintained at 180 °F (82.2 °C). The spray drying resulted in a galacto-oligosaccharide powder ("GOS powder").

### EXAMPLE 4

The same permeate feedstock as in Examples 1-3 was subjected to reverse osmosis to provide a permeate having a solids content of 20% TS.

The 20% TS permeate was sent through a nanofiltration membrane sold under the trade name SR3D membrane by Koch Membrane Systems Inc., Wilmington, MA, USA. The nanofiltration membrane was designed for selective rejection of salts from the fluid being fed to the nanofiltration membrane. The nanofiltration membrane was made from a thin-film composite (TFC) polyamide and had a molecular weight cutoff of 200 Daltons. The nanofiltration membrane was in the form of a spiral-bound filter with a net outer wrap. The nanofiltration membrane had an inner diameter of 4 inches and an outer diameter of 8 inches. The nanofiltered permeate had a reduced salt content compared to the feedstock permeate and the 20% TS permeate (discussed in more detail below), and is hereinafter referred to as a low-salt permeate.

The low-salt permeate was further concentrated by evaporation to provide a concentrated low-salt permeate having a solids content of 40% TS. The 40 % TS low-salt permeate was exposed to an enzyme mixture of the β-galactosidase from *Aspergillus oryzae* and the β-galactosidase from *Kluveromyces lactis.* The enzymes were hydrated with potable process water at a temperature of about 70 °F (about 21.1 °C) to about 80 °F (about 26.7 °C). The enzyme concentration after mixing the enzyme mixture with the 40% TS low-salt permeate was about 0.01% to about 0.05% by weight. The 40% TS low-salt permeate was allowed to react with the enzyme mixture for about 2 hours to about 4 hours at a temperature of from about 125 °F (51.7 °C) to about 135 °F (57.2 °C). The enzyme treatment resulted in a galacto-oligosaccharide solution having a reduced salt content ("low-salt GOS solution") with a solids content of about 40% TS.

The low-salt GOS solution was further concentrated by evaporation to provide a galacto-oligosaccharide syrup having a reduced salt content ("low-salt GOS syrup") with a solids content of about 60% to about 70% TS. The evaporation to form the low-salt GOS syrup also deactivated the enzymes from the enzyme mixture.

The low-salt GOS syrup was then dried in a spray dryer at a feed concentration of about 40% TS. The inlet temperature of the spray dryerwas held at about 240 °F (115.6 °C). The outlet temperature of the spray dryer was maintained at 180 °F (82.2 °C). The spray drying resulted in a galacto-oligosaccharide powder having a reduced salt content ("low-salt GOS powder").

### EXAMPLE 5

The same permeate feedstock as in Examples 1-4 was subjected to reverse osmosis to provide a permeate having a solids content of 20% TS. The 20% TS permeate was then further concentrated by evaporation to provide a concentrated permeate having a solids content of 40% TS.

The 40% TS permeate was exposed to an enzyme mixture of the β-galactosidase from *Aspergillus oryzae* and the β-galactosidase from *Kluveromyces lactis.* The enzymes were hydrated with potable process water at a temperature of about 70 °F (about 21.1 °C) to about 80 °F (about 26.7 °C). The enzyme concentration after mixing the enzyme mixture with the 40% TS permeate was about 0.01% to about 0.05% by weight. The 40% TS permeate was allowed to react with the enzyme mixture for about 2 hours to about 4 hours at a temperature of from about 125 °F (51.7 °C) to about 135 °F (57.2 °C). The enzyme treatment resulted in a galacto-oligosaccharide solution ("GOS solution") having a solids content of about 40% TS.

The GOS solution was sent through a nanofiltration membrane sold under the trade name SR3D membrane by Koch Membrane Systems Inc., Wilmington, MA, USA. The nanofiltration membrane was designed for selective rejection of salts, glucose, and galactose from the fluid being fed to the nanofiltration membrane. The nanofiltration membrane was made from a thin-film composite (TFC) polyamide and had a molecular weight cutoff of 200 Daltons. The nanofiltration membrane was in the form of a spiral-bound filter with a net outer wrap. The nanofiltration membrane had an inner diameter of 4 inches and an outer diameter of 8 inches. The nanofiltered GOS solution had a reduced salt content, a reduced glucose content, and a reduced galactose content compared to the feedstock permeate and the GOS solution, and is hereinafter referred to as a "low salt/glucose/galactose GOS solution." The low-salt/glucose/galactose GOS solution was further concentrated by evaporation to provide a concentrated low-salt/glucose/galactose syrup having a solids content of about 60% to about 70% TS.

The low-salt/glucose/galactose GOS syrup was dried in a spray dryer at a feed concentration of about 40% TS. The inlet temperature of the spray dryer was held at about 240 °F (115.6 °C). The outlet temperature of the spray dryer was maintained at 180 °F (82.2 °C). The spray drying resulted in a galacto-oligosaccharide powder having a reduced salt content, reduced glucose content, and reduced galactose content ("low-salt/glucose/galactose GOS powder").

### DISCUSSION OF EXAMPLES 1-5

The composition of the 60 % TS GOS syrup batch, the 70% TS GOS syrup batch, and the 80% TS GOS gel from Example 1 are provided in Table 1.

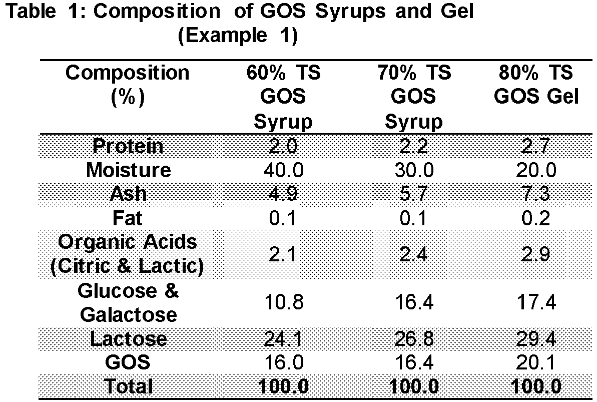

Details of the composition of the GOS powder produced by Examples 2-5 are shown in Tables 2 and 3. Table 2 shows the dry basis content of the main constituent categories (e.g., GOS, glucose, galactose, lactose, ash, protein, fat, salt, organic acids, and moisture) in the GOS powders from Examples 2 and 3, the low-salt GOS powder from Example 4, and the low-salt/glucose/galactose GOS powder from Example 5. Table 3 shows the content of several common minerals for the GOS powders from Examples 2 and 3, the low-salt GOS powder from Example 4, and the low-salt/glucose/galactose GOS powder from Example 5. For comparison, Tables 2 and 3 also provide the dry basis composition of the initial permeate feedstock for the processes of Examples 1-5.

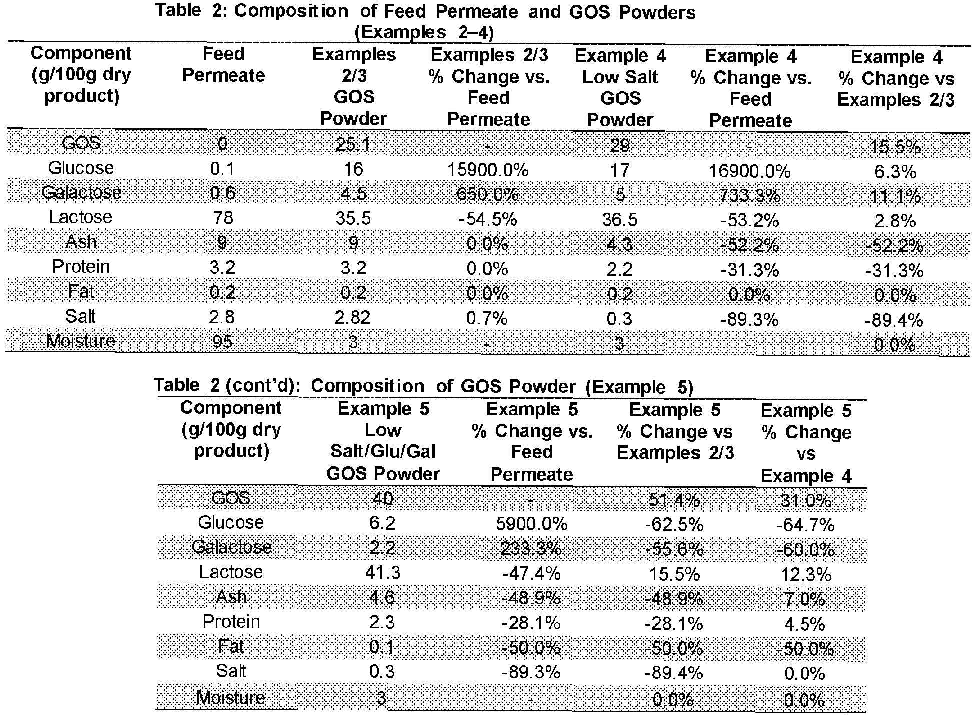

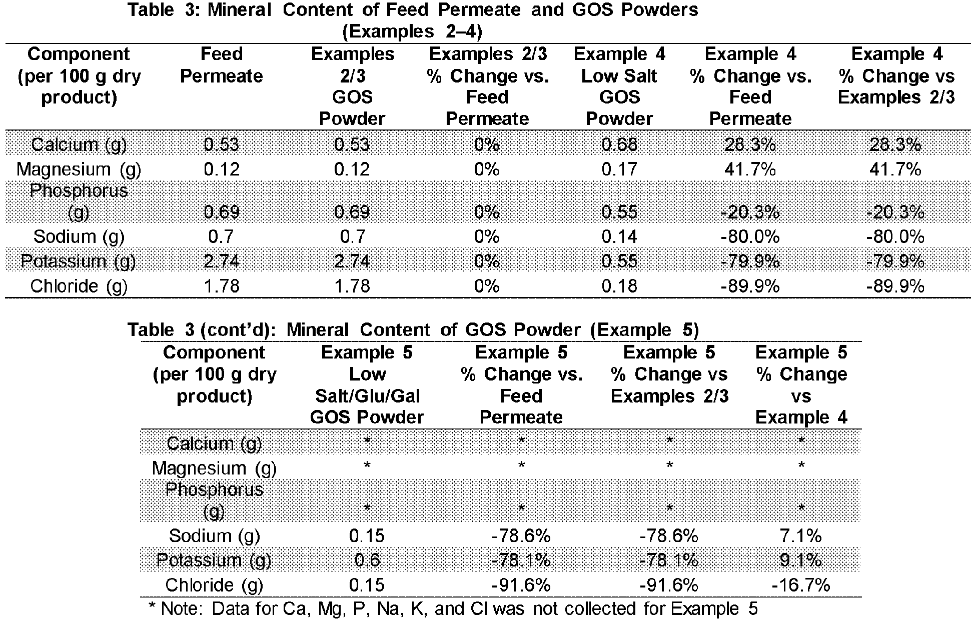

The compositions of the GOS products (e.g., the GOS syrups and the GOS gel produced in Example 1 or by the process 100, e.g., as provided in Table 1, and of the GOS powders from Examples 2-5 and processes 200, 300, and 400) are different from current commercially available GOS products. For example, ash and sugars present in the GOS syrups, the GOS gel, and the GOS powders can be utilized by the entire digestive system and can provide advantageous properties for growth, activity, and nutrition. For example, the chloride present in the GOS products (e.g., shown for the GOS powders in Table 2, but also present in the syrups and gel in Example 1) can be used in the production of hydrochloric acid in the stomach, which can be necessary for digestion. Sodium ions present in the GOS products (see Table 2) can provide for absorption of glucose and amino acids in the small intestine. Calcium and magnesium present in the GOS products (see Table 2) can be absorbed by the large intestine. In some examples, the galacto-oligosaccharide compounds present in the GOS products can be utilized by *bifidobacterium* microorganisms in the intestines. For example, the processes described herein can result in the GOS products being comprised of mostly trisaccharides and oligosaccharides that have been shown to enhance the growth of *bifidobacterium* microorganisms in the intestine. As such, in some examples, the GOS products described herein can provide a strong enhancement of the growth of *bifidobacteria*, such as in human or animal intestines, which is also referred to as a strong "bifidus factor."

In an example, the nanofiltration to provide a low-salt permeate that is subjected to enzyme treatment, as in Example 4, can result in a GOS product (e.g., the low-salt GOS powder of Example 4 or process 300) with an increased galacto-oligosaccharide content after the enzyme treatment compared to GOS products from a comparable process without the nanofiltration (e.g., the GOS powder from the processes of Examples 2 and 3 or process 200). For example, as shown in Table 2, the nanofiltration before the enzyme treatment, as in Example 4, results in a low-salt GOS powder that is about 29 g GOS per 100 g of dry product, compared to the comparable GOS powder from Examples 2 and 3 that do not include nanofiltration, which resulted in a GOS content of about 25 g per 100 g of product, or an increase of about 15%. In an example, the nanofiltration before the enzyme treatment can also result in slight increases in glucose (e.g., from about 16 g/100 g dry product for Examples 2 and 3 to about 17 g/100 g for Example 4, or about a 6.25% increase, in Table 2), galactose (e.g., from about 4.5 g/100 g for Examples 2 and 3 to about 5 g/100 g for Example 4, or about a 11% increase, in Table 2), and lactose (e.g., from about 35.5 g/100 g dry product for Examples 2 and 3 to about 36.5 g/100 g dry product for Example 4, or about 2.8%, in Table 2). However, the increases in these other sugars are smaller when compared to GOS, and therefore may not be statistically significant, particularly with respect to lactose and glucose. The increase in GOS, glucose, galactose, and lactose may simply be due to the decrease in salts, ash, and other components, i.e., not due to an increase in formation of the sugars by the one or more enzymes. However, the increases in glucose, galactose, and lactose could also be due to the removal of salts and other components resulting in improved conversion to GOS and other sugars by the one or more enzymes because of reduce concentrations of one or more of salts, ash, and other components.

In addition to the nutritional and digestive benefits described above with respect to the GOS products, the low-salt GOS products described herein (e.g., the low-salt GOS powder of Example 4 and process 300) can have nutritional advantages for infant nutrition or for those with high blood pressure or other physical conditions that are benefited by a low-salt diet.

In an example, the nanofiltration after the enzyme treatment, as in Example 5 and process 400, can result in a GOS product with an increased galacto-oligosaccharide content compared to GOS products from a process without the nanofiltration, e.g., as in Examples 1-3 and processes 100 and 200. For example, as shown in Table 2, the nanofiltration after the enzyme treatment can result in a GOS product (e.g., the low-salt/glucose/galactose GOS powder from Example 5) that is about 38 g GOS per 100 g of dry product, compared to a comparable GOS product from a process without nanofiltration (e.g., the GOS powders from Examples 2 and 3), which resulted in a GOS content of about 25 g per 100 g of product, or an increase of about 51%. In an example, the nanofiltration after the enzyme treatment can also result in a GOS product (e.g., the low-salt/glucose/galactose GOS powder of Example 5) with a higher GOS content than a comparable GOS product from a process with nanofiltration before the enzyme treatment (e.g., the low-salt GOS powder from Example 4). For example, as shown in Table 2, the low-salt/glucose/galactose GOS powder from Example 5, resulting from nanofiltration after the enzyme treatment, has a GOS content of about 38 g/100 g of dry product, compared to the 29 g/100 g dry product for the low-salt GOS powder of Example 4, an increase of about 31%. The increase in GOS content is likely due to the reduction of monosaccharides (e.g., glucose and galactose) between the two GOS products.

In an example, the nanofiltration after the enzyme treatment, as in Example 5 and process 400, can also result in a slight increase in lactose compared to processes without nanofiltration, as in Examples 2 and 3 and process 200. For example, as shown in Table 2, the lactose increased from about 35.5 g/100 g dry product in Examples 2 and 3 to about 41 g/100 g for Example 5, or about a 15.5% increase, in Table 2. The nanofiltration after the enzyme treatment can also result in slight increases, compared to a process with nanofiltration before the enzyme treatment (e.g., Example 4 and process 300), for lactose (e.g., from about 36.5 g/100 g for Example 4 to about 41 g/100 g for Example 5, or about a 12% increase, in Table 2), ash (e.g., from about 4.3 g/100 g dry product for Example 4 to about 4.6 g/100 g dry product for Example 5, or about a 7% increase, in Table 2), protein (e.g., from about 2.2 g/100 g dry product for Example 4 to about 2.3 g/100 g dry product for Example 5), and organic acids (e.g., from about 2.5 g/100 g dry product for Example 4 to about 2.7 g/100 g dry product for Example 2, or about an 8% increase). However, the increases in these other components are smaller when compared to GOS, and therefore may not be statistically significant.

In addition to the nutritional and digestive benefits described above with respect to the other GOS products described herein, the low-salt, low-glucose, and low-galactose GOS products described herein (e.g., the low salt/glucose/galactose powder of Example 5 and of the process 400) can be used as a low-glycemic index ingredient for food products, such as a low-glycemic index sweetener.

### EXAMPLE 6

The same permeate feedstock as Examples 1-5 was subjected to reverse osmosis to provide a permeate having a solids content of 20% TS. The 20% TS permeate was then further concentrated by evaporation to provide a concentrated permeate having a solids content of 45% TS.

The 45% TS permeate was exposed to a β-galactosidase enzyme from *Aspergillus oryzae.* The enzyme was hydrated with potable process water at a temperature of about 70 °F (about 21.1 °C) to about 80 °F (about 26.7 °C). The enzyme concentration after mixing the enzyme with the 45% TS permeate was about 0.01% to about 0.05% by weight. The 45% TS permeate was allowed to react with the enzyme for about 4 hours to about 5 hours at a temperature of from about 130 °F (54.4 °C) to about 140 °F (60.0 °C). The enzyme treatment resulted in a galacto-oligosaccharide solution ("GOS solution") having a solids content of about 45% TS. The enzyme in the 45% TS GOS solution was deactivated by heating the GOS solution to a temperature above 175 °F (about 79.4 °C).

The GOS solution was then dried in a spray dryer at a feed concentration of about 45% TS. The inlet temperature of the spray dryerwas held at about 300 °F (148.9 °C). The outlet temperature of the spray dryer was maintained at about 180 °F (82.2 °C). The spray drying resulted in a galacto-oligosaccharide powder ("EXAMPLE 6 GOS Powder"), having a GOS content of about 30.6% on a weight-to-weight basis. The spray dryerwas able to dry the GOS solution to a flowable, non-tacky powder without the use of any added drying agent, i.e., without the use of milk protein, caseinate, whey protein, nonfat dry milk, skim milk, lactose, tricalcium phosphate, dicalcium phosphate, kaolin, diatomaceous earth, silica, calcium silicate hydrate, maltodextrin, starches, or mixtures thereof.

### EXAMPLE 7

The same permeate feedstock as Example 1 was subjected to reverse osmosis to provide a permeate having a solids content of 20% TS. The 20% TS permeate was then further concentrated by evaporation to provide a concentrated permeate having a solids content of 45% TS.

The 45% TS permeate was exposed to a β-galactosidase enzyme from *Aspergillus oryzae.* The enzyme was hydrated with potable process water at a temperature of about 70 °F (about 21.1 °C) to about 80 °F (about 26.7 °C). The enzyme concentration after mixing the enzyme with the 45% TS permeate was about 0.01% to about 0.05% by weight. The 45% TS permeate was allowed to react with the enzyme for about 4 hours at a temperature of from about 130 °F (54.4 °C) to about 140 °F (60.0 °C). The enzyme treatment resulted in a galacto-oligosaccharide solution ("GOS solution") having a solids content of about 45% TS. The enzyme in the 45% TS GOS solution was deactivated by heating the GOS solution to a temperature above 175 °F (about 79.4 °C).

The 45% TS GOS solution was then further concentrated by evaporation to provide a galacto-oligosaccharide syrup ("GOS syrup"), having a solids content of about 60% TS.

The 60% TS GOS syrup was then dried in a spray dryer at a feed concentration of about 60% TS. The inlet temperature of the spray dryerwas held at about 300 °F (148.9 °C). The outlet temperature of the spray dryer was maintained at about 180 °F (82.2 °C). The spray drying resulted in a galacto-oligosaccharide powder ("EXAMPLE 7 GOS Powder"), having a GOS content of about 26.5% on a weight-to-weight basis. The spray dryerwas able to dry the GOS solution to a flowable, non-tacky powder without the use of any added drying agent, i.e., without the use of milk protein, caseinate, whey protein, nonfat dry milk, skim milk, lactose, tricalcium phosphate, dicalcium phosphate, kaolin, diatomaceous earth, silica, calcium silicate hydrate, maltodextrin, starches, or mixtures thereof.

### DISCUSSION OF EXAMPLES 6 AND 7

Samples of the EXAMPLE 6 GOS powder and the EXAMPLE 7 GOS powder were analyzed to determine a detailed compositional breakdown of the monosaccharides and oligosaccharides, as well as other compounds present in the samples. The inventors found that it was very difficult to analyze the samples via analytical techniques that are typically used for analyzing dairy-based or other food-based products. Consultation was therefore sought from the Complex Carbohydrate Research Center at the University of Georgia, whose research is supported by the Chemical Sciences, Geosciences and Biosciences Division of the Office of Basic Energy Sciences, U.S. Department of Energy Grant Number DE-SC0015662 to Parastoo Azadi at the Complex Carbohydrate Research Center. However, the United States government does not have any rights in the present application.

### TOTAL CARBOHYDRATE ANALYSIS

Samples of the EXAMPLE 6 GOS Powder and the EXAMPLE 7 GOS Powder were analyzed to determine the total sugar/carbohydrate amount for each sample. To do so, a 2.41 milligram (mg) sample of the EXAMPLE 6 GOS Powder and a 2.31 mg of the EXAMPLE 7 GOS Powder were each dissolved in 800 microliters (µl) of a 2 N solution of trifluoracetic acid (TFA) and each sample was allowed to hydrolyze at 121 °C for two (2) hours. After hydrolysis, the resulting digest solution was dried under a stream of nitrogen gas, then evaporated two times with isopropanol and was then dissolved in water (H₂O).

For example sample, known amounts of glucose and galactose monosaccharide standards were hydrolyzed in the same manner and at the same time as the sample. Five concentrations of the standard mixture were prepared to establish a calibration curve. The quantity of each residue in the sample was calculated by linear interpolation of respective residue area units into the calibration equation.

Monosaccharides were analyzed by high-performance anion exchange chromatography with pulsed amperometric detection ("HPAEC-PAD") using an ICS-3000 ion chromatogray system sold by Dionex Corp. of Sunnyvale, California, USA (now sold by ThermoFisher Scientific Inc. of Waltham, Massachusetts, USA). The Dionex ICS-3000 system was equipped with a gradient pump, an electrochemical detector, and an autosampler. Glycosyl residues were separated by a CarboPac PA20 (3x 150 mm) analytical column, also sold by Dionex Corp. (now part of ThermoFisher Scientific Inc.), with a pre-installed amino trap and eluted with degassed nanopure water and 200 millimolar (mM) NaOH. Injection was made every 43 minutes.

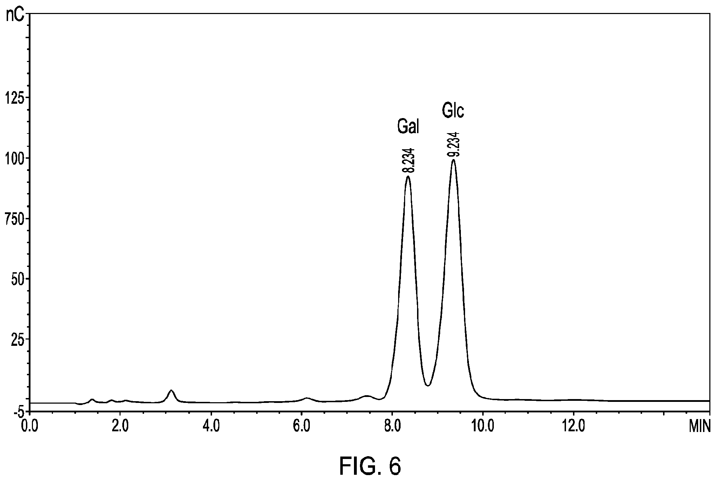

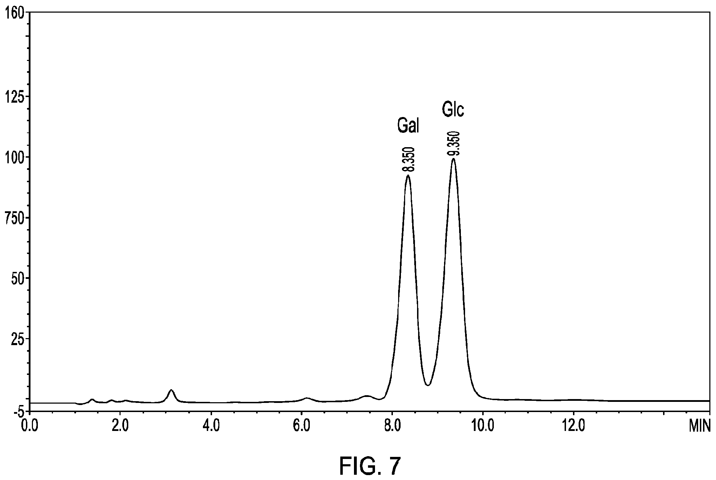

FIGS. 6 and 7 are profile graphs for the monosaccharides after HPAEC-PAD analysis of the hydrolyzed samples of the GOS Powders. FIG. 6 shows the HPAEC for the EXAMPLE 6 GOS Powder sample (labeled as "GOS 5" in FIG. 6), and FIG. 7 shows the HPAEC for the EXAMPLE 7 GOS Powder sample (labeled as "GOS 9" in FIG. 7). Table 4 summarizes the monosaccharide composition analysis by HPAEC-PAD:

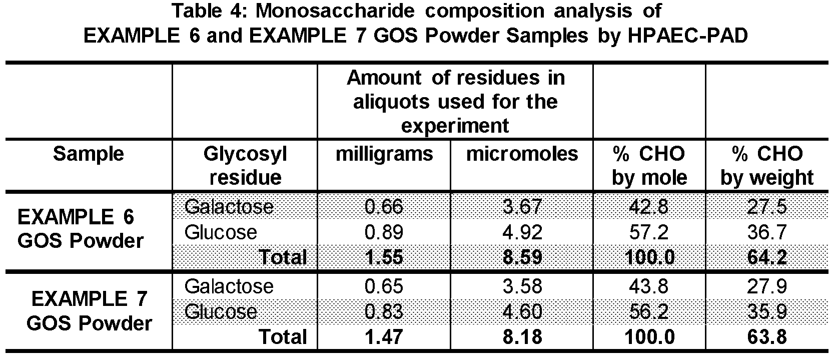

The glycosyl composition analysis of FIGS. 6 and 7 and in Table 4 does not include the absolute configuration (D- or L-) of the monosaccharides. The data from the hydrolyzed whole samples in Table 4 are the total of free residues plus residues released from oligomeric and polymeric structures after hydrolysis.

As is shown in Table 4, the EXAMPLE 6 GOS Powder had a total carbohydrate content, by weight, of about 64.2 wt.% and the EXAMPLE 7 GOS Powder had a total carbohydrate content, by weight, of about 63.8 wt.%. In other words, the HPAEC-PAD analysis of the GOS Powders found that the EXAMPLE 6 GOS Powder was 64.2% carbohydrate, by weight and that the EXAMPLE 7 GOS Powder was 63.8% carbohydrate, by weight.

### CARBOHYDRATE BREAKDOWN ANALYSIS

Samples of the EXAMPLE 6 GOS Powder and the EXAMPLE 7 GOS Powder were also analyzed to determine the breakdown of sugars and carbohydrates, e.g., to determine the amount of galacto-oligosaccharides present in each GOS Powder. For the purposes of this analysis, "galacto-oligosaccharides" or "GOS" will be defined as an oligosaccharide compound made up of two or more monosaccharide units (i.e., with a degree of polymerization that is ≥2) wherein at least one of the monosaccharide units comprises a galactose group, except that lactose (i.e., the disaccharide comprising a galactose monosaccharide and a glucose monosaccharide joined by a β-1,4 glycosidic bond) will not be considered a GOS for the purposes of this analysis. The degree of polymerization ("DP") of the compound can be anywhere from 2 to about 20, such as from 2 to about 15, for example from 2 to about 10. However, in many examples, GOS compounds will have a DP of from 2 (designated as "DP2") to 8 (designated as "DP8"). As can be seen in the analysis below, the particular samples of GOS Powders that resulted from EXAMPLE 6 and EXAMPLE 7 only included GOS compounds of DP7 or less.

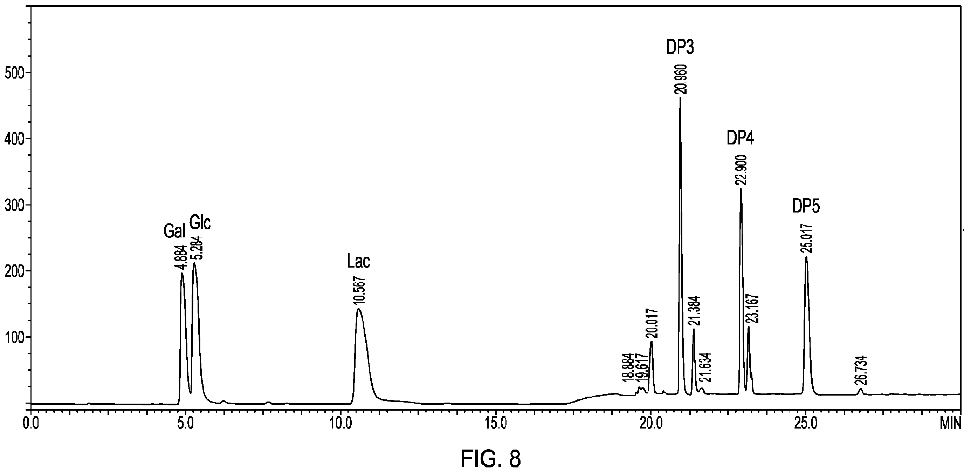

To analyze the specific oligosaccharide breakdown of the GOS Powders, standard solutions were prepared, including a glucose (Glc) solution, a galactose (Gal) solution, a lactose (Lac) solution, a maltotriose solution, a maltotetraose solution, and a maltopentaose solution, were prepared, with each standard compound having a concentration in its solution of 0.05 µg/µl. The standard solutions were analyzed by HPAEC using the Dionex ICS-3000 system described above, equipped with a gradient pump, an electrochemical detector, and an autosampler. The individual mono- and oligosaccharides mixture were separated by a Dionex CarboPac PA200 (3 x 250 mm) analytical column with PA200 guard column. The gradient program used the following mobile phase eluents: nanopure water and 500 mM sodium hydroxide and 0.5M sodium acetate in 500 mM NaOH. Injection was made every 45 min. The methods were based on protocols described by Hardy, M. R., and Townsend, R. R. in "High-pH anion-exchange chromatography of glycoprotein-derived carbohydrates," 1994, Methods Enzymol. 230, at pages 208-25. FIG. 8 shows the HPAEC profile that resulted from the HPAEC analysis of the standards solution sample

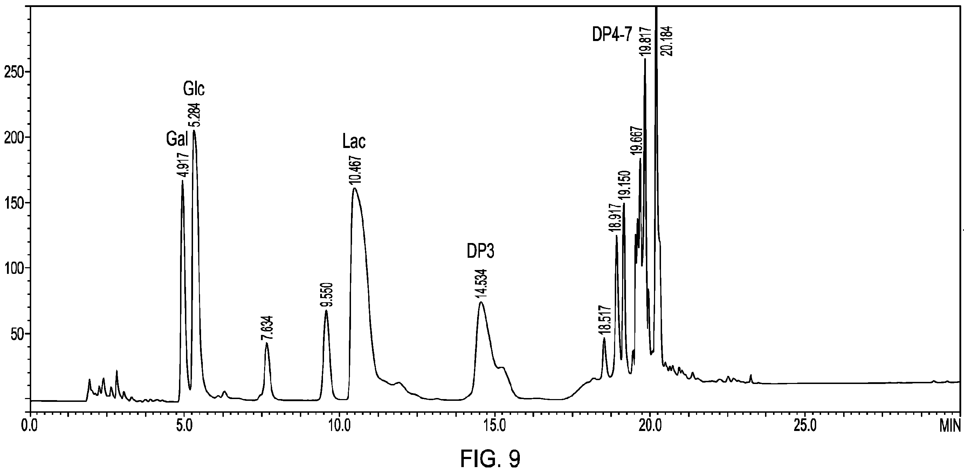

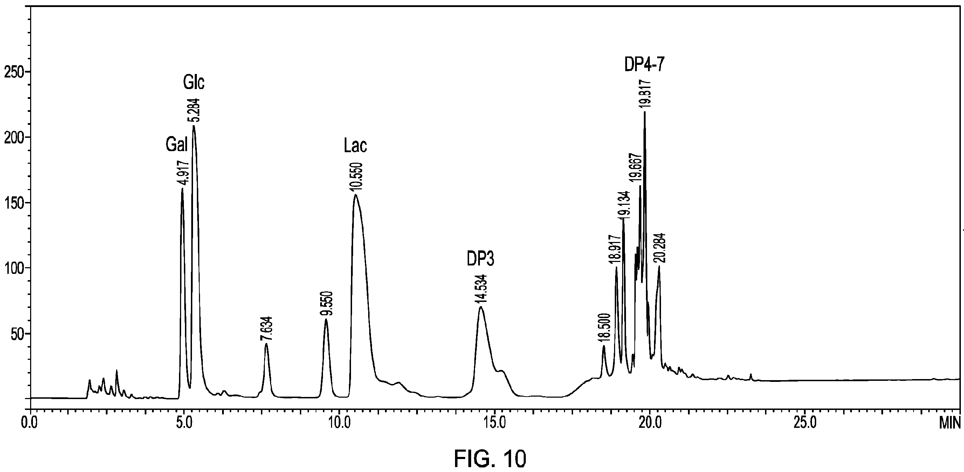

An aqueous solution was prepared from the EXAMPLE 6 GOS Powder ("EXAMPLE 6 Solution Sample") and a separate solution was prepared from the EXAMPLE 7 GOS Powder ("EXAMPLE 7 Solution Sample"). Each of the EXAMPLE 6 Solution Sample and the EXAMPLE 7 Solution Sample were prepared at a concentration of 0.05 µg/µl so that they were comparable to the standard compounds in the standard solution, described above. Ten (10) µl of each of the EXAMPLE 6 Solution Sample and the EXAMPLE 7 Solution Sample were injected onto the HPAEC column. The Samples were analyzed by HPAEC under the same conditions as described above for the standards solution. FIGS. 9 and 10 show the HPAEC profiles that resulted from the HPAEC analysis of the EXAMPLE 6 Solution Sample and the EXAMPLE 7 Solution Sample, respectively.

In the HPAEC analysis, the standards for galactose, glucose, lactose and the malto-oligomers (DP3-DP5) were run on the PA200 column and were separated well, as can be seen by the distinct peaks in FIG. 8. In the analysis of the EXAMPLE 6 GOS Powder and the EXAMPLE 7 GOS Powder, galactose, glucose, and lactose (DP2) were identified fairly readily by the retention time (RT) as their peaks corresponded substantially with the peaks from the galactose, glucose, and lactose standards (see the comparison of the standards of FIG. 8 with the EXAMPLE 6 and EXAMPLE 7 Samples in FIGS. 9 and 10, respectively). However, DP3-DP7 oligomer peaks of both the EXAMPLE 6 Sample (FIG. 9) and the EXAMPLE 7 Sample (FIG. 10) did not separate as well as the standard malto-oligomers. This suggests that the oligomers in the GOS samples are different from the malto-standards.

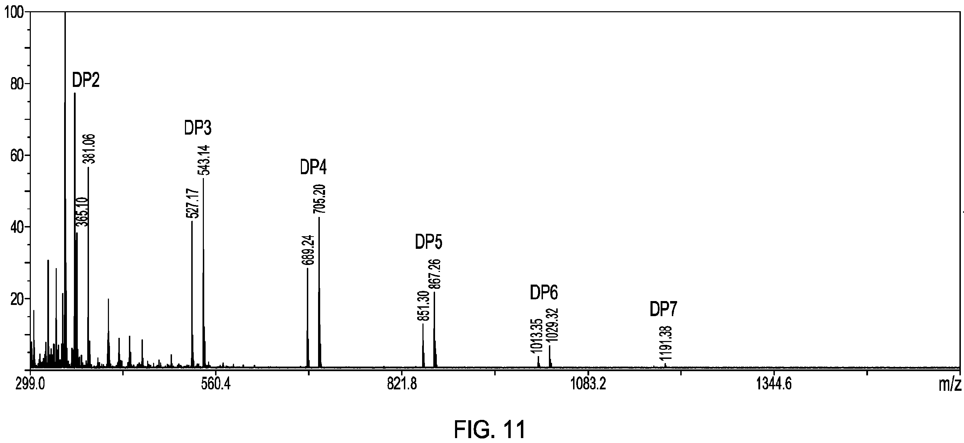

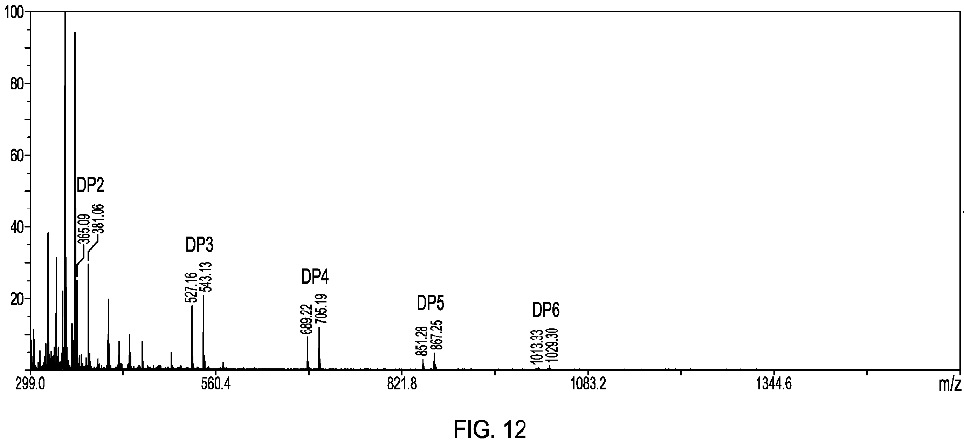

MALDI-TOF mass spectrometry was therefore performed to further detect hexose-oligomers with DP3-DP7. Additional samples of the EXAMPLE 6 GOS Powder and of the EXAMPLE 7 GOS Powder were separately dissolved in deionized water, with each solution having a concentration of one (1) mg/ml. One (1) µl of each solution was separately mixed with one (1) µl of 2,5-dihydroxybenzoic acid matrix (DHBA) to form a second EXAMPLE 6 Solution Sample and a second EXAMPLE 7 Solution. MALDI-TOF mass spectrometry was performed on each of the second EXAMPLE 6 Solution Sample and the second EXAMPLE 7 Solution Sample. FIGS. 11 and 12 show the profiles that resulted from the MALDI-TOF MS analysis of the second EXAMPLE 6 Solution Sample and the second EXAMPLE 7 Solution Sample, respectively. HPAEC peak assignments were made according to the residence time (RT) after the glucose and galactose monosaccharides.

Table 5 shows an analysis of the area under the peaks of the HPAEC profiles (in units of nanocoulomb*minutes (nC*min)) corresponding to the separate components found in the EXAMPLE 6 GOS Powder and the EXAMPLE 7 GOS Powder, i.e., for DP1 monosaccharides (galactose and glucose), DP2 disaccharides (including lactose and non-lactose disaccharides), DP3 trisaccharides, DP4 tetrasaccharides, D5 oligosaccharides, D6 oligosaccharides, and D7 oligosaccharides. Relative peak areas were calculated from the integrated peak areas using software sold under the trade name CHROMELEON by the Thermo Fisher Scientific Inc., Waltham, Massachusetts, USA. As noted above, the GOS Powders produced in Examples 6 and 7 did not produce oligosaccharides of DP8 or higher. Despite this, the methods and systems of the present application are not limited to D7 oligosaccharides or lower.

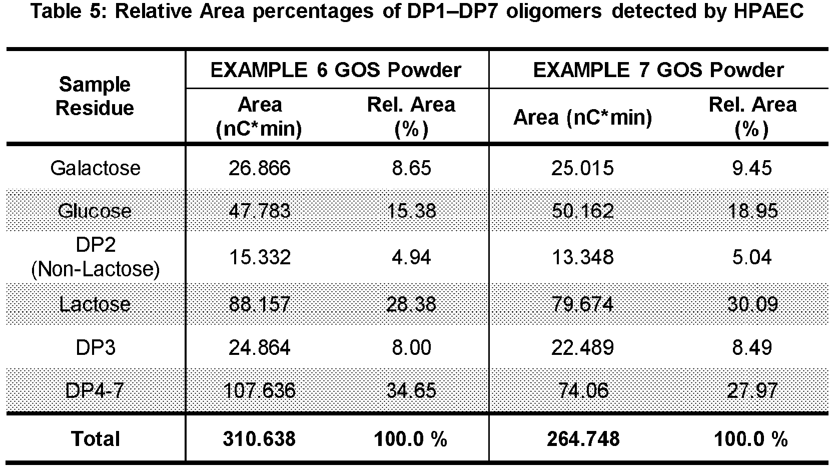

The relative area under the peaks for a particular class of saccharide compound directly corresponds to the relative weight percentage of that class of saccharide compound compared to all of the classes of saccharide compounds measured by the HPAEC analysis. Therefore, the values in the Relative Area column in Table 5 are assumed to be equal to the weight% relative to the total carbohydrates in each GOS Powder sample. For example, the weight% of DP3 oligosaccharides relative to the total carbohydrates in the EXAMPLE 6 GOS Powder is about 8.0% (w/w), while galactose is about 8.65% (w/w).

As noted above, Table 4 shows the data corresponding to the HPAEC-PAD analysis to determine the total carbohydrate composition of the GOS Powders, which found that the EXAMPLE 6 GOS Powder had a total carbohydrate content of about 64.2 wt.% and the EXAMPLE 7 GOS Powder had a total carbohydrate content of about 63.8 wt.%. This result from Table 4 can be combined with the relative carbohydrate % breakdown from Table 5 to provide an estimated absolute weight percentage of each class of carbohydrate in each GOS Powder. For example, the EXAMPLE 6 GOS Powder was found to be 64.2 wt.% carbohydrate in Table 4, and Table 5 shows that the relative percentage galactose in the EXAMPLE 6 GOS Powder was 8.65%, so that the overall weight percentage of galactose is calculated as (64.2 wt.% total carbohydrate) x (8.65 wt.% galactose/wt.% carbohydrates = about 5.6 wt.% galactose in the EXAMPLE 6 GOS Powder. Similarly, the EXAMPLE 7 GOS Powder was found to be 63.8 wt.% carbohydrate in Table 4, and Table 5 shows the relative percentage of galactose in the EXAMPLE 7 GOS Powder was 9.45%, resulting in the overall weight percentage of galactose being calculated as (63.8 wt.% carbohydrate) x (9.45 wt.% glucose/wt.% carbohydrate) = about 6.0 wt.% glucose in the EXAMPLE 7 GOS Powder. The result of these calculations for each of the other residue components from Table 5 are shown in Table 6.

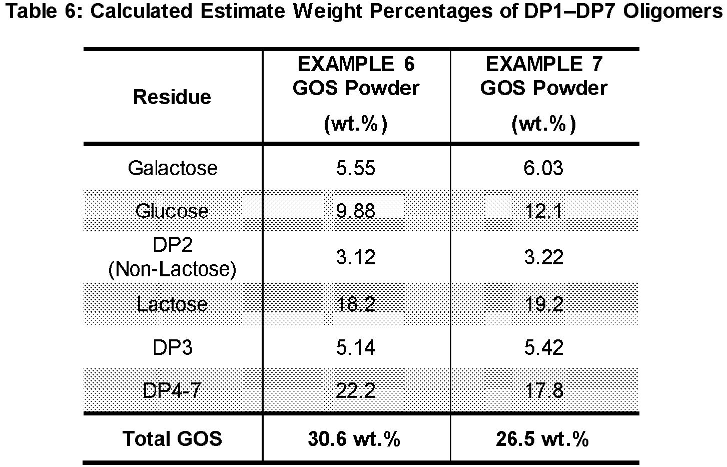

As noted above, for the purposes of this analysis, GOS is defined as any DP2 disaccharide that includes galactose except for lactose, and any D3 or greater oligosaccharide that includes galactose. Because of the specific enzymes used to form the compounds in the EXAMPLE 6 and EXAMPLE 7 GOS Powders, it is assumed that all or substantially of the non-lactose DP2 and DP3 or greater oligosaccharides include at least one galactose monosaccharide unit, such that the total weight percentage of GOS compounds in each GOS Powder is equal to the sum of the non-lactose DP2 compounds, the D3 oligosaccharide compounds, and the D4-D7 oligosaccharide compounds, as shown in Table 5.

The above Detailed Description is intended to be illustrative, and not restrictive. For example, the above-described examples (or one or more elements thereof) can be used in combination with each other. Other embodiments can be used, such as by one of ordinary skill in the art upon reviewing the above description. Also, various features or elements can be grouped together to streamline the disclosure. This should not be interpreted as intending that an unclaimed disclosed feature is essential to any claim. Rather, inventive subject matter can lie in less than all features of a particular disclosed embodiment. Thus, the following claims are hereby incorporated into the Detailed Description, with each claim standing on its own as a separate embodiment. The scope of the invention should be determined with reference to the appended claims, along with the full scope of equivalents to which such claims are entitled.

In the event of inconsistent usages between this document and any documents so incorporated by reference, the usage in this document controls.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc., are used merely as labels, and are not intended to impose numerical requirements on their objects.

Method examples described herein can be machine or computer-implemented, at least in part. Some examples can include a computer-readable medium or machine-readable medium encoded with instructions operable to configure an electronic device to perform methods or method steps as described in the above examples. An implementation of such methods or method steps can include code, such as microcode, assembly language code, a higher-level language code, or the like. Such code can include computer readable instructions for performing various methods. The code may form portions of computer program products. Further, in an example, the code can be tangibly stored on one or more volatile, non-transitory, or non-volatile tangible computer-readable media, such as during execution or at other times. Examples of these tangible computer-readable media can include, but are not limited to, hard disks, removable magnetic disks, removable optical disks (e.g., compact disks and digital video disks), magnetic cassettes, memory cards or sticks, random access memories (RAMs), read only memories (ROMs), and the like.

## Claims

1. A process for producing a galacto-oligosaccharide product, the process comprising:
exposing a permeate composition comprising a whey permeate, a milk permeate, or a mixture of a whey and milk permeate to one or more enzymes, wherein the permeate composition comprises a minimum lactose content of 76 wt%;
wherein the one or more enzymes convert one or more compounds in the permeate composition to one or more galacto-oligosaccharides to provide a galacto-oligosaccharide solution, wherein at least 10% of total sugar, by weight, in the galacto-oligosaccharide solution is in the form of the one or more galacto-oligosaccharides;
concentrating at least a portion of the galacto-oligosaccharide solution to provide a galacto-oligosaccharide syrup; and
drying the galacto-oligosaccharide syrup to provide a flowable powder without the use of a drying agent compound.

2. The process according to claim 1, wherein the powder comprises at least 20 wt% of the one or more galacto-oligosaccharide compounds.

3. The process according to any one of claims 1-2, further comprising crystallizing at least a portion of the galacto-oligosaccharide syrup.

4. The process according to any one of claims 1-3, wherein the one or more enzymes comprise at least one of a β-galactosidase from the fungus *Aspergillus oryzae* or a β-galactosidase from the yeast *Kluveromyces lactis.*

5. The process according to any one of claims 1-4, wherein the permeate composition has a solids content of at least 20%, by weight, total solids when it is exposed to the one or more enzymes.

6. The process according to any of claims 1-5, wherein the permeate composition has a solids content of at least 50%, by weight, total solids when it is exposed to the one or more enzymes.

7. The process according to any one of claims 1-6, further comprising concentrating one or more permeate byproducts to provide the permeate composition, wherein the one or more permeate byproducts comprise a whey permeate byproduct, a milk permeate byproduct, or a mixture of a whey permeate byproduct and a milk permeate byproduct.

8. The process according to any one of claims 1-7, further comprising deactivating the one or more enzymes after a specified time period.

9. The process according to any one of claims 1-8, wherein the permeate composition consists essentially of the whey permeate, the milk permeate, or the mixture of whey permeate and milk permeate.

10. The process according to any one of claims 1-9, wherein the permeate composition has a solids content of from 20% to 50%, by weight, total solids when exposed to the one or more enzymes.

11. The process according to any one of claims 1-10, wherein the permeate composition has a solids content of from 30% to 40%, by weight, total solids when exposed to the one or more enzymes.

12. The process according to any one of claims 1-11, wherein from 10% to 50% of the total sugar, by weight, in the galacto-oligosaccharide solution is in the form of the one or more galacto-oligosaccharides.

13. The process according to any one of claims 1-12, wherein the flowable powder comprises at least 30 wt% of the one or more galacto-oligosaccharides.

## Patentansprüche

1. Verfahren zur Herstellung eines Galacto-Oligosaccharid-Produkts, wobei das Verfahren umfasst:
Aussetzen einer Permeatzusammensetzung, die ein Molkepermeat, ein Milchpermeat oder eine Mischung aus einer Molke und einem Milchpermeat umfasst, einem oder mehreren Enzymen, wobei die Permeatzusammensetzung einen minimalen Lactosegehalt von 76 Gew.-% umfasst;
wobei das eine oder die mehreren Enzyme eine oder mehrere Verbindungen in der Permeatzusammensetzung in ein oder mehrere Galacto-Oligosaccharide umwandeln, um eine Galacto-Oligosaccharid-Lösung bereitzustellen, wobei mindestens 10% des Gesamtzuckers, gewichtsbezogen, in der Galacto-Oligosaccharid-Lösung in Form des einen oder der mehreren Galacto-Oligosaccharide vorliegen;
Konzentrieren mindestens eines Teils der Galacto-Oligosaccharid-Lösung, um einen Galacto-Oligosaccharid-Sirup bereitzustellen; und
Trocknen des Galacto-Oligosaccharid-Sirups, um ein fließfähiges Pulver ohne die Verwendung einer Trockenmittelverbindung bereitzustellen.

2. Verfahren nach Anspruch 1, wobei das Pulver mindestens 20 Gew.-% der einen oder mehreren Galacto-Oligosaccharid-Verbindungen umfasst.

3. Verfahren nach einem der Ansprüche 1-2, weiter umfassend das Kristallisieren mindestens eines Teils des Galacto-Oligosaccharid-Sirups.

4. Verfahren nach einem der Ansprüche 1-3, wobei das eine oder die mehreren Enzyme mindestens eines von einer β-Galactosidase aus dem Pilz *Aspergillus oryzae* oder einer β-Galactosidase aus der Hefe *Kluveromyces lactis* umfassen.

5. Verfahren nach einem der Ansprüche 1-4, wobei die Permeatzusammensetzung einen Feststoffgehalt von mindestens 20%, gewichtsbezogen, Gesamtfeststoffgehalt aufweist, wenn sie dem einen oder den mehreren Enzymen ausgesetzt wird.

6. Verfahren nach einem der Ansprüche 1-5, wobei die Permeatzusammensetzung einen Feststoffgehalt von mindestens 50%, gewichtsbezogen, Gesamtfeststoffgehalt, aufweist, wenn sie dem einen oder den mehreren Enzymen ausgesetzt wird.

7. Verfahren nach einem der Ansprüche 1-6, weiter umfassend das Konzentrieren eines oder mehrerer Permeatnebenprodukte, um die Permeatzusammensetzung bereitzustellen, wobei das eine oder die mehreren Permeatnebenprodukte ein Molkepermeatnebenprodukt, ein Milchpermeatnebenprodukt oder eine Mischung eines Molkepermeatnebenprodukts und eines Milchpermeatnebenprodukts umfassen.

8. Verfahren nach einem der Ansprüche 1-7, weiter umfassend das Deaktivieren des einen oder der mehreren Enzyme nach einem bestimmten Zeitraum.

9. Verfahren nach einem der Ansprüche 1-8, wobei die Permeatzusammensetzung im Wesentlichen aus dem Molkepermeat, dem Milchpermeat oder der Mischung aus Molkepermeat und Milchpermeat besteht.

10. Verfahren nach einem der Ansprüche 1-9, wobei die Permeatzusammensetzung einen Feststoffgehalt von 20% bis 50%, gewichtsbezogen, Gesamtfeststoffgehalt aufweist, wenn sie dem einen oder den mehreren Enzymen ausgesetzt wird.

11. Verfahren nach einem der Ansprüche 1-10, wobei die Permeatzusammensetzung einen Feststoffgehalt von 30% bis 40%, gewichtsbezogen, Gesamtfeststoffgehalt aufweist, wenn sie dem einen oder den mehreren Enzymen ausgesetzt wird.

12. Verfahren nach einem der Ansprüche 1-11, wobei 10% bis 50% des Gesamtzuckers, gewichtsbezogen, in der Galacto-Oligosaccharid-Lösung in Form des einen oder der mehreren Galacto-Oligosaccharide vorliegen.

13. Verfahren nach einem der Ansprüche 1-12, wobei das fließfähige Pulver mindestens 30 Gew.-% des einen oder der mehreren Galacto-Oligosaccharide umfasst.

## Revendications

1. Procédé de production d'un produit de galacto-oligosaccharide, le procédé comprenant :
une exposition d'une composition de perméat comprenant un perméat de lactosérum, un perméat de lait ou un mélange d'un perméat de lactosérum et de lait à une ou plusieurs enzymes, dans lequel la composition de perméat comprend une teneur en lactose minimale de 76 % en poids ;
dans lequel les une ou plusieurs enzymes convertissent un ou plusieurs composés dans la composition de perméat en un ou plusieurs galacto-oligosaccharides pour fournir une solution de galacto-oligosaccharide, dans lequel au moins 10 % de sucre total, en poids, dans la solution de galacto-oligosaccharide est sous la forme des un ou plusieurs galacto-oligosaccharides ;
une concentration au moins d'une partie de la solution de galacto-oligosaccharide pour fournir un sirop de galacto-oligosaccharide ; et
un séchage du sirop de galacto-oligosaccharide pour fournir une poudre fluide sans l'utilisation d'un composé d'agent de séchage.

2. Procédé selon la revendication 1, dans lequel la poudre comprend au moins 20 % en poids des un ou plusieurs composés de galacto-oligosaccharide.

3. Procédé selon l'une quelconque des revendications 1-2, comprenant en outre une cristallisation au moins d'une partie du sirop de galacto-oligosaccharide.

4. Procédé selon l'une quelconque des revendications 1-3, dans lequel les une ou plusieurs enzymes comprennent au moins l'une d'une β-galactosidase provenant du champignon Aspergillus oryzae ou une β-galactosidase provenant de la levure Kluyveromyces lactis.

5. Procédé selon l'une quelconque des revendications 1-4, dans lequel la composition de perméat présente une teneur en solides d'au moins 20 % en poids de solides totaux lorsqu'elle est exposée aux une ou plusieurs enzymes.

6. Procédé selon l'une quelconque des revendications 1-5, dans lequel la composition de perméat présente une teneur en solides d'au moins 50 % en poids de solides totaux lorsqu'elle est exposée aux une ou plusieurs enzymes.

7. Procédé selon l'une quelconque des revendications 1-6, comprenant en outre une concentration d'un ou plusieurs produits dérivés de perméat pour fournir la composition de perméat, dans lequel les un ou plusieurs produits dérivés de perméat comprennent un produit dérivé de perméat de lactosérum, un produit dérivé de perméat de lait, ou un mélange d'un produit dérivé de perméat de lactosérum et d'un produit dérivé de perméat de lait.

8. Procédé selon l'une quelconque des revendications 1-7, comprenant en outre une désactivation des une ou plusieurs enzymes après une période de temps spécifiée.

9. Procédé selon l'une quelconque des revendications 1-8, dans lequel la composition de perméat consiste essentiellement en le perméat de lactosérum, le perméat de lait, ou le mélange de perméat de lactosérum et de perméat de lait.

10. Procédé selon l'une quelconque des revendications 1-9, dans lequel la composition de perméat présente une teneur en solides de 20 % à 50 % en poids de solides totaux lorsqu'elle est exposée aux une ou plusieurs enzymes.

11. Procédé selon l'une quelconque des revendications 1-10, dans lequel la composition de perméat présente une teneur en solides de 30 % à 40 % en poids de solides totaux lorsqu'elle est exposée aux une ou plusieurs enzymes.

12. Procédé selon l'une quelconque des revendications 1-11, dans lequel de 10 % à 50 % du sucre total, en poids, dans la solution de galacto-oligosaccharide est sous la forme des un ou plusieurs galacto-oligosaccharides.

13. Procédé selon l'une quelconque des revendications 1-12, dans lequel la poudre fluide comprend au moins 30 % en poids des un ou plusieurs galacto-oligosaccharides.
